(19) Europäisches Patentamt

European Patent Office

Office européen des brevets

(11) **EP 1 283 044 A2**

(12) **EUROPEAN PATENT APPLICATION**

(43) Date of publication:
**12.02.2003 Bulletin 2003/07**

(51) Int Cl.[7]: **A61K 31/704**, A61P 35/00

(21) Application number: **02025856.2**

(22) Date of filing: **03.12.1987**

(84) Designated Contracting States:
**AT BE CH DE ES FR GB GR IT LI LU NL SE**

(30) Priority: **05.12.1986 GB 8629193**
**22.06.1987 US 64653**

(62) Document number(s) of the earlier application(s) in accordance with Art. 76 EPC:
**95101919.9 / 0 659 435**
**91102986.6 / 0 438 183**

(71) Applicant: **Pharmacia Italia S.p.A.**
**20152 Milano (IT)**

(72) Inventors:
• **Gatti, Gaetano**
**Seto San Giovani, Milan (IT)**
• **Oldani, Diego**
**Robecco sul Naviglio, Milan (IT)**

• **Bottoni, Giuseppe**
**24100 Bergamo (IT)**
• **Confalonieri, Carlo**
**Cusano Milanino (Milan) (IT)**
• **Gambini, Luciano**
**Cornaredo (Milan) (IT)**
• **De Ponti, Roberto**
**Milan (IT)**

(74) Representative: **Woods, Geoffrey Corlett**
**J.A. KEMP & CO.,**
**Gray's Inn,**
**14 South Square**
**London WC1R 5LX (GB)**

Remarks:
This application was filed on 19 - 11 - 2002 as a divisional application to the application mentioned under INID code 62.

(54) **Injectable ready-to-use solutions containing an antitumor anthracycline glycoside**

(57) A storage stable, sterile, pyrogen-free, injectable anthracycline glycoside solution consists essentially of a physiologically acceptable salt of an anthracycline glycoside dissolved in a physiologically acceptable solvent therefor, which has not been reconstituted from a lyophilizate and the pH of which has been adjusted to from 2.5 to 4.0

**EP 1 283 044 A2**

Printed by Jouve, 75001 PARIS (FR)

## Description

The present invention relates to a storage stable, injectable ready-to-use solution of an antitumor anthracycline glycoside, e.g. doxorubicin, and to a process for preparing such a solution.

The anthracycline glycoside compounds are a well known class of compounds in the antineoplastic group of agents, of which doxorubicin is a typical, and the most widely used, representative: Doxorubicin. Anticancer Antibiotics, Federico Arcamone, 1981, Publ: Academic Press, New York, N.Y.; Adriamycin Review, EROTC International Symposium, Brussels, May, 1974, edited by M. Staquet, Publ. Eur. Press Medikon, Ghent, Belg.; and Results of Adriamycin Therapy, Adriamycin Symposium at Frankfurt/Main 1974 edited by M.Ghione, J.Fetzer and H.Maier, publ.: Springer, New York, N.Y.

At present, anthracycline glycoside antitumor drugs, in particular, e.g. doxorubicin, are solely available in the form of lyophilized preparations, which need to be reconstituted before administration. Both the manufacture and the reconstitution of such preparations expose the involved personnel (workers, pharmacists, medical personnel, nurses) to risks of contamination which are particularly serious due to the toxicity of the antitumor substances.

The Martindale Extra Pharmacopoeia 28th edition, page 175 left column, reports on adverse effects of antineoplastic drugs and recommends that "They must be handled with great care and contact with skin and eyes avoided; they should not be inhaled. Care must be taken to avoid extravasation since pain and tissue damage may ensue.". Similarly, Scand. J. Work Environ Health vol.10 (2), pages 71-74 (1984), as well as articles in Chemistry Industry, Issue July 4, 1983, page 488, and Drug-Topics-Medical-Economics-Co, Issue February 7, 1983, page 99 report about severe adverse effects observed in medical personnel exposed to use of cytostatic agents, including doxorubicin.

To administer a lyophilized preparation, double handling of the drug is required. The lyophilized cake first has to be reconstituted and then administered. Moreover, in some cases, the complete dissolution of the powder may require prolonged shaking because of solubilization problems. Reconstitution of a lyophilized cake or powder can result in formation of aerosol droplets which can be inhaled or can come into contact with skin or mucous membranes of those handling the solution.

As the risks connected with the manufacture and the reconstitution of a lyophilized preparate would be highly reduced if a ready-to-use solution of the drug were available, we have developed a stable, therapeutically acceptable intravenously injectable solution of an anthracycline glycoside drug, e.g. doxorubicin, whose preparation and administration does not require either lyophilization or reconstitution.

GB-A-2178311 describes and claims sterile, pyrogen-free, anthracycline glycoside solutions which consist essentially of a physiologically acceptable salt of an anthracycline glycoside such as doxorubicin dissolved in a physiologically acceptable solvent therefor, which have not been reconstituted from a lyophilizate and which have a pH of from 2.5 to 6.5. Especially preferred pH's are about 3 and about 5. The Examples illustrate solutions with pH's ranging from 2.62 to 3.14, with pH 4.6 and with pH 5.2. There is no mention of stabilising agents. Dextrose, lactose and mannitol are mentioned as tonicity adjustment agents but no indication is given of the proportions in which they may be used.

We have now found that such solutions have a better stability when they possess a pH of from 2.5 to 4.0. Accordingly, the present invention provides a storage-stable sterile, pyrogen-free, injectable anthracycline glycoside solution which consists essentially of a physiologically acceptable salt of an anthracycline glycoside dissolved in a physiologically acceptable solvent therefor, which has not been reconstituted from a lyophilizate and the pH of which has been adjusted to from 2.5 to 4.0.

It is thus possible to provide solutions which are storage stable and have a commercially meaningful shelf-life.

Preferably the solution of the invention is provided in a sealed container, especially one made of glass. The solution can be provided in this way either in a unit dosage form or in a multiple dosage form.

Preferably the anthracycline glycoside is chosen from doxorubicin, 4'-epi-doxorubicin (i.e. epirubicin), 4'-desoxy-doxorubicin (i.e. esorubicin), 4'-desoxy-4'-iodo-doxorubicin, daunorubicin and 4-demethoxy-daunorubicin (i.e. idarubicin). A particularly preferred anthracycline glycoside is doxorubicin.

Any physiologically acceptable salt of the anthracycline glycoside may be used for preparing the solution of the invention. Examples of suitable salts may be, for instance, the salts with mineral inorganic acids such as hydrochloric, hydrobromic, sulfuric, phosphoric, nitric and the like, and the salts with certain organic acids such as acetic, succinic, tartaric, ascorbic, citric, glutamic, benzoic, methanesulfonic, ethanesulfonic and the like. The salt with hydrochloric acid is a particularly preferred salt, especially when the anthracycline glycoside is doxorubicin.

Any solvent which is physiologically acceptable and which is able to dissolve the anthracycline glycoside salt may be used. The solution of the invention may also contain one or more formulation adjuvants such as a co-solubilizing agent (which may be the same as a solvent), a stabilising agent, a tonicity adjustment agent, a preservative and a pharmaceutically acceptable chelating agent.

Suitable solvents and co-solubilizing agents may be, for instance, water e.g. Water for Injections; a 0.9% sodium chloride solution, i.e. physiological saline; an aqueous 5% dextrose solution; aliphatic amides, e.g. N,N-dimethylacetamide, N-hydroxy-2-ethyl-lactamide and the like; alcohols, e.g. ethanol, benzyl alcohol and the like; glycols and polyalcohols, e.g. propyleneglycol, glycerin and the like; esters of polyalcohols, e.g. diacetine., triacetine and the like; polyglycols and polyethers, e.g. polyethyleneglycol 400, propyleneglycol methylethers and the like; dioxolanes, e.g. isopropylidenglycerin and the like; dimethylisosorbide; pyrrolidone derivatives, e.g. 2-pyrrolidone, N-methyl-2-pyrrolidone, polyvinylpyrrolidone (co-solubilizing agent only) and the like; polyoxyethylenated fatty alcohols, e.g. Brij (trade mark) and the like; esters of polyoxyethylenated fatty acids, e.g. Cremophor (trade mark), Myrj (trade mark) and the like; polysorbates, e.g. Tweens (trade mark); polyoxyethylene derivatives of polypropyleneglycols, e.g. Pluronics (trade mark).

Examples of preferred solvents are water, physiological saline, an aqueous 5% dextrose solution, ethanol, polyethyleneglycol and dimethylacetamide as well as mixtures in various proportions of these solvents. Water, physiological saline and a 5% dextrose solution are particularly preferred.

A particularly preferred co-solubilizing agent is polyvinylpyrrolidone.

A sugar or sugar alcohol may be present as a stabilising agent. Suitable stabilizing agents include saccharides such as monosaccharides (e.g. dextrose, galactose, fructose and fucose), disaccharides (e.g. lactose), polysaccharides, (e.g. dextran) and cyclic oligosaccharides (e.g. $\alpha$-, $\beta$- or $\gamma$-cyclodextrin); and mono- or poly-hydric aliphatic alcohols including cyclic polyols (e.g. mannitol, sorbitol, glycerol, thioglycerol, ethanol and inositol). The stabilizing agent may be included in concentrations of from about 0.25 to 10% w/v, preferably from 0.5 to 5% w/v in the solution.

Sugars and sugar alcohols may also serve as tonicity adjustment agents. Suitable tonicity adjustment agents may be, for instance, physiologically acceptable inorganic chlorides, e.g. sodium chloride (preferably 0.9% by weight sodium chloride), dextrose, lactose, mannitol, sorbitol and the like. When a sugar or sugar alcohol such as dextrose, lactose mannitol or sorbitol is present, preferably from 2.5 to 7.5% by weight is used, more preferably from 4 to 6% by weight and most preferably about 5% by weight.

Preservatives suitable for physiological administration may be, for instance, esters of para-hydroxybenzoic acid (e.g., methyl, ethyl, propyl and butyl esters, or mixtures of them), chlorocresol and the like.

A suitable pharmaceutically acceptable chelating agent may be ethylenediaminotetraacetic acid (EDTA). The chelating agent is included in a minor amount, typically from 0.001 to 0.05 % by weight.

The above mentioned solvents and co-solubilizing agents, stabilizing agents, tonicity adjustment agents, preservatives and chelating agents can be used alone or as a mixture of two or more of them.

To adjust the pH within the range of from 2.5 to 4.0 a physiologically acceptable acid or buffer is added as desired. The acid may be any physiologically acceptable acid, e.g. an inorganic mineral acid such as hydrochloric, hydrobromic, sulfuric, phosphoric, nitric and the like, or an organic acid such as acetic, succinic, tartaric, ascorbic, citric, glutamic, benzoic, methanesulphonic, ethanesulfonic and the like, or also an acidic physiologically acceptable buffer solution, e.g., a chloride buffer, a glycine buffer, a formate buffer, an acetate buffer, a phosphate buffer and the like.

The preferred range of pH for the ready-to-use solution of the invention is from 2.5, e.g. from about 2.6, to about 3.7, e.g. to about 3.5. A more preferred pH range is from about 3 to about 3.5. A pH of about 3 is particularly preferred especially where the solution of the invention contains sorbitol or from 2.5 to 7.5 % by weight of dextrose, lactose or mannitol. Other preferred pH ranges are from greater than 3.14, e.g. from about 3.2, to 4 and preferably to about 3.7, more preferably to about 3.5. A useful solution with a pH of from 2.62 to 3.14 further comprises:

(i) from 0.25 to 10% w/v of a stabilizing and/or tonicity adjustment agent selected from saccharides and mono- and poly-hydric aliphatic alcohols with the proviso that dextrose, mannitol or lactose, when present as a tonicity adjustment agent, is present in an amount of from 2.5 to 7.5% by weight; and/or
(ii) a pharmaceutically acceptable chelating agent.

In the solutions of the invention the concentration of the anthracycline glycoside may vary within broad ranges, preferably from 0.1 mg/ml to 100 mg/ml, in particular from 0.1 mg/ml to 50 mg/ml, most preferably from 1 mg/ml to 20 mg/ml.

The preferred ranges of concentration may be slightly different for different anthracycline glycosides. Thus, for example, preferred concentrations for doxorubicin are from about 2 mg/ml to about 50 mg/ml, preferably from 2 mg/ml to 20 mg/ml, particularly appropriate values being 2 mg/ml and 5 mg/ml. Similar concentrations are preferred also for 4'-epi-doxorubicin, 4'-desoxy-doxorubicin and 4'-desoxy-4'-iodo-doxorubicin. Preferred ranges of concentration for daunorubicin and 4-demethoxy-daunorubicin are from 0.1 mg/ml to 50 mg/ml, preferably from 1 mg/ml to 20 mg/ml, concentrations of 1 mg/ml and 5 mg/ml being particularly appropriate.

Suitable packaging for the anthracycline glycoside solutions may be all approved containers intended for parenteral use, such as plastic and glass containers, ready-to-use syringes and the like. Preferably the container is a sealed glass container, e.g. a vial or an ampoule. A hermetically sealed glass vial is particularly preferred.

The invention also provides a process for producing a storage stable, sterile, pyrogen-free, injectable, anthracycline glycoside solution according to the invention, which process comprises

(i) dissolving the physiologically acceptable salt of the anthracycline glycoside, which salt is not in the form of a lyophilizate, in the physiologically acceptable solvent therefor;

(ii) optionally adding the one or more formulation adjuvants selected from co-solubilizing agents, stabilising agents, tonicity adjustment agents, preservatives and pharmaceutically acceptable chelating agents; and

(iii) adding a physiologically acceptable acid or buffer to adjust the pH to from 2.5 to 4.0 as desired; the process being effected in such a manner that the resultant solution is sterile and pyrogen-free.

Any suitable procedure may be adopted to ensure that the resultant solution is sterile and pyrogen-free. Preferably, the solution is passed through a sterilising filter after step (iii) although of course one or more of the materials used may be sterile and pyrogen-free anyway. Where all materials employed are sterile and pyrogen-free, there may then be no need for passing the resultant solution through a sterilising filter.

With the solutions of the invention it is possible to obtain compositions having a very high concentration of the anthracycline glycoside active substance even at 50 mg/ml and more. This constitutes a great advantage over the presently available lyophilized preparates wherein high concentrations of anthracycline glycoside can only be obtained with difficulty because of solubilization problems encountered in reconstitution, mainly with saline. The presence of the excipient, e.g. lactose, in the lyophilized cake, and its generally high proportion in respect of the active substance, even up to 5 parts of excipient per part of active substance, has a negative effect on solubilization so that difficulties may arise in obtaining dissolution of the lyophilized cake, especially for concentrations of anthracycline glycoside higher than 2 mg/ml.

The solutions of the invention are characterized by a good stability. Solutions in various solvents and with different pH's and concentrations have been found to be stable for long periods at temperatures accepted for the storage of pharmaceutical preparations. This is illustrated in the Examples which follow.

Owing to the well known anti-tumor activity of the anthracycline glycoside active drug substance, the pharmaceutical compositions of the invention are useful for treating tumors in both human and animal hosts. Examples of tumors that can be treated are, for instance, sarcomas, including osteogenic and soft tissue sarcomas, carcinomas, e.g., breast-, lung-, bladder-, thyroid-, prostate- and ovarian carcinoma, lymphomas, including Hodgkin and non-Hodgkin lymphomas, neuroblastoma, melanoma, myeloma, Wilms tumor, and leukemias, including acute lymphoblastic leukemia and acute myeloblastic leukemia.

Examples of specific tumours that can be treated are Moloney Sarcoma Virus, Sarcoma 180 Ascites, solid Sarcoma 180, gross transplantable leukemia, L 1210 leukemia and lymphocytic P 388 leukemia.

Inhibition of the growth of a tumour, in particular one of those indicated above, can be achieved by administering to a host suffering from a said tumour an injectable solution according to the invention containing the active drug substance in an amount sufficient to inhibit the growth of said tumour.

The injectable solutions of the invention are administered by rapid e.g. intravenous injection or infusion according to a variety of possible dose schedules.

A Suitable dose schedule for doxorubicin may be, for example, of 60 to 75 mg of active drug substance per $m^2$ of body surface given as a single rapid infusion and repeated at 21 days. An alternative schedule may be of 30 mg/$m^2$ day be intravenous route for 3 days, every 28 days. Suitable dosages for 4'-epi-doxorubicin and 4'-desoxy-doxorubicin may be, for instance, of 75 to 90 mg/$m^2$ given in a single infusion to be repeated at 21 days, and similar dosages may be useful also for 4'-desoxy-4'-iodo-doxorubicin.

Idarubicin, i.e. 4-demethoxy-daunorubicin, may be, e.g. administered intravenously at a single dose of 13-15 mg/$m^2$ every 21 days in the treatment of solid tumours, while in the treatment of leukemias a preferred dose schedule is, e.g., of 10-12 ng/$m^2$ day by intravenous route for 3 days, to be repeated every 15-21 days. Similar dosages may be, e.g., followed for daunorubicin.

The following Examples illustrate the invention. In the accompanying drawings; in which "E + 3" means " x $10^3$" and "d" means "days":

Figure 1 is the $K_{obs}$-pH profile for the doxorubicin.HCl degradation at 55°C in sterile water (Example 1);

Figure 2 is the $K_{obs}$-pH profile for the doxorubicin.HCl degradation at 55°C in 5% dextrose (Example 1);

Figure 3 is the $K_{obs}$-pH profile for the doxorubicin.HCl degradation at 55°C in 0.9% (physiological) saline (Example 1);

Figure 4 is the $K_{obs}$-pH profile for the epirubicin.HCl degradation at 55°C in sterile water (Water for Injections) (Example 7);

Figure 5 is the $K_{obs}$-pH profile for the epirubicin.HCl degradation at 55°C in 0.9% (physiological) saline (Example 7); and

Figure 6 is the $K_{obs}$-pH profile for the epirubicin.HCl degradation at 55°C in 5% dextrose (Example 7).

Example 1: pH-stability profile at 55°C of doxorubicin.HCl solutions in sterile water, 5% dextrose or 0.9% saline

Doxorubicin.HCl was dissolved at a concentration of 2 mg/ml in the following I=0.05 buffers:

(a) glycine.HCl pH 2.0, 2.5 and 3.0;
(b) formate pH 3.5; and
(c) acetate pH 4.0, 5.0 and 5.5.

Each solution was filtered through a 0.22 µm microporous membrane under nitrogen pressure. 5.0 ml of each solution were stored at 55°C in glass vials of glass type I, 8ml top capacity vial, Teflon (Trade Mark)-faced chlorobutyl rubber bung, aluminium seal. Each solution was analysed at predetermined times (up to 120 hours) for doxorubicin. HCl assay and pH. The results are shown in Tables 1, 2 and 3 which give the doxorubicin.HCl residual concentration and percent stability at 55°C, at different pHs and times of storage for sterile water, 5% dextrose and 0/9% saline solutions, respectively.

The doxorubicin.HCl assays are the mean of three independent determinations performed by the US Pharmacopoeia (USP high performance liquid chromatography (HPLC) method (USP XXI)). At each pH value, the pseudo-first order rate constants ($K_{obs}$) for the degradation were calculated by linear regression analysis of the natural logarithm of the residual concentration of doxorubicin.HCl ($|Dx|_t$) versus time as depicted by the following equation:

$$\ln |Dx|_t = \ln |Dx|_o - K_{obs} \cdot t$$

Tables 4, 5 and 6 give the observed rate constants ($K_{obs}$) for the degradation kinetics of doxorubicin.HCl at 55°C and at different pHs for sterile water, 5% dextrose and 0.9% saline solutions, respectively. Figures 1, 2 and 3 of the accompanying drawings show the $K_{obs}$ - pH profile for the doxorubicin.HCl degradation at 55°C in the above mentioned media.

The data in Tables 1 - 6 and the Figures 1 - 3 evidence that the 2 mg/ml doxorubicin.HCl solutions show at 55°C good stability at pH 2.5 - 4.0 and maximum stability at pH 3.0 - 3.5 for all three media tested. A common behaviour as to stability is thus evidenced for aqueous solutions in general, since no practical differences in stability are observed when passing from sterile water as such to sterile water containing a tonicity adjustment agent, either ionic, such as, e.g., sodium chloride, or non-ionic, such as, e.g., dextrose.

Table 1-

| Accelerated (55°C) stability data of 2 mg/ml doxorubicin·HCl solutions in sterile water at various pHs | | | | | | | | |
|---|---|---|---|---|---|---|---|---|
| Buffers | Tests | Time (hours) | | | | | | |
| | | 0 | 8 | 16 | 24 | 48 | 72 | 120 |
| pH 2.0 glycine-HCl | Doxorubicin·HCl assay | | | | | | | |
| | . mg/ml | 2.022 | 1.892 | 1.669 | 1.554 | 1.145 | 0.801 | |
| | . % stability | 100.0 | 93.6 | 82.6 | 76.9 | 56.6 | 39.6 | |
| | pH | 2.00 | 2.01 | 2.02 | 2.01 | 2.01 | 2.02 | |
| pH 2.5 glycine-HCl | Doxorubicin·HCl assay | | | | | | | |
| | . mg/ml | 1.992 | 1.926 | 1.835 | 1.718 | 1.557 | 1.00 | |
| | . % stability | 100.0 | 96.7 | 92.1 | 86.2 | 78.2 | 50.2 | |
| | pH | 2.51 | 2.50 | 2.50 | 2.52 | ·2.51 | 2.52 | |
| pH 3.0 glycine-HCl | Doxorubicin·HCl assay | | | | | | | |
| | . mg/ml | 2.003 | 1.958 | 1.881 | 1.831 | 1.696 | 1.525 | 1.258 |
| | . % stability | 100.0 | 97.8 | 93.9 | 91.4 | 84.7 | 76.1 | 62.8 |
| | pH | 3.00 | 3.03 | 3.02 | 3.02 | 3.01 | 3.02 | 3.00 |

Table 1- (continued)

| Accelerated (55°C) stability data of 2 mg/ml doxorubicin·HCl solutions in sterile water at various pHs | | | | | | | | |
|---|---|---|---|---|---|---|---|---|
| Buffers | Tests | Time (hours) | | | | | | |
| | | 0 | 8 | 16 | 24 | 48 | 72 | 120 |
| pH 3.5 formate | Doxorubicin·HCl assay | | | | | | | |
| | . mg/ml | 2.035 | 1.950 | 1.887 | 1.840 | 1.650 | 1.538 | 1.241 |
| | . % stability | 100.0 | 95.8 | 92.7 | 90.4 | 81.1 | 75.6 | 61.0 |
| | pH | 3.51 | 3.51 | 3.51 | 3.51 | 3.52 | 3.52 | 3.51 |
| pH 4.0 acetate | Doxorubicin·HCl assay | | | | | | | |
| | . mg/ml | 2.032 | 1.788 | 1.681 | 1.561 | 1.167 | | |
| | . % stability | 100.0 | 88.0 | 82.7 | 76.8 | 57.4 | | |
| | pH | 4.00 | 4.00 | 4.04 | 4.02 | 4.02 | | |
| pH 5.0 acetate | Doxorubicin·HCl assay | | | | | | | |
| | . mg/ml | 2.019 | 1.823 | 1.688 | 1.512 | 1.060 | | |
| | . % stability | 100.0 | 90.3 | 83.6 | 74.9 | 52.5 | | |
| | pH | 5.03 | 5.05 | 5.04 | 5.04 | 5.05 | | |
| pH 5.5 acetate | Doxorubicin·HCl assay | | | | | | | |
| | . mg/ml | 2.047 | 1.808 | 1.427 | 1.228 | 0.903 | | |
| | . % stability | 100.0 | 88.3 | 69.7 | 60.0 | 84.1 | | |
| | pH | 5.50 | 5.53 | 5.53 | 5.51 | 5.56 | | |

Table 2 - Accelerated (55°C) stability data of 2 mg/ml doxorubicin·HCl solutions in 5% dextrose at various pHs

| Buffers | Tests | Time (hours) | | | | | | | | |
|---|---|---|---|---|---|---|---|---|---|---|
| | | 0 | 8 | 16 | 24 | 34 | 48 | 72 | 96 | 120 |
| pH 2.0 glycine-HCl | Doxorubicin·HCl assay | | | | | | | | | |
| | . mg/ml | 1.993 | 1.851 | 1.683 | 1.513 | 1.361 | 1.078 | 0.765 | | |
| | . % stability | 100.0 | 92.8 | 84.4 | 75.9 | 68.3 | 54.1 | 38.4 | | |
| | pH | 2.14 | 2.13 | 2.14 | 2.15 | 2.18 | 2.21 | 2.16 | | |
| pH 2.5 glycine-HCl | Doxorubicin·HCl assay | | | | | | | | | |
| | . mg/ml | 1.967 | 1.897 | 1.822 | 1.760 | 1.682 | 1.499 | 1.305 | | |
| | . % stability | 100.0 | 96.4 | 92.6 | 89.5 | 85.5 | 76.2 | 66.3 | | |
| | pH | 2.56 | 2.56 | 2.56 | 2.58 | 2.60 | 2.56 | 2.61 | | |
| pH 3.0 glycine-HCl | Doxorubicin·HCl assay | | | | | | | | | |
| | . mg/ml | 1.975 | | 1.908 | 1.832 | | 1.645 | 1.508 | 1.344 | 1.206 |
| | . % stability | 100.0 | | 96.6 | 92.7 | | 83.3 | 76.4 | 68.0 | 61.1 |
| | pH | 3.04 | | 3.05 | 3.05 | | 3.06 | 3.00 | 3.13 | 3.10 |
| pH 3.5 formate | Doxorubicin·HCl assay | | | | | | | | | |
| | . mg/ml | 1.983 | | 1.897 | 1.858 | | 1.622 | 1.324 | 1.222 | |
| | .% stability | 100.0 | | 95.7 | 93.7 | | 81.8 | 66.8 | 61.6 | |
| | pH | 3.58 | | 3.59 | 3.60 | | 3.63 | 3.60 | 3.63 | |
| pH 4.0 acetate | Doxorubicin·HCl assay | | | | | | | | | |
| | . mg/ml | 2.003 | 1.913 | 1.716 | 1.665 | 1.487 | 1.312 | 1.081 | | |
| | . % stability | 100.0 | 95.5 | 85.6 | 83.1 | 74.2 | 65.5 | 53.9 | | |
| | pH | 4.10 | 4.10 | 4.11 | 4.11 | 4.16 | 4.15 | 4.12 | | |
| pH 5.0 acetate | Doxorubicin·HCl assay | | | | | | | | | |
| | . mg/ml | 2.012 | 1.906 | 1.673 | 1.608 | 1.416 | 1.163 | | | |
| | . % stability | 100.0 | 94.7 | 83.2 | 79.9 | 70.4 | 57.8 | | | |
| | pH | 5.06 | 5.06 | 5.06 | 5.06 | 5.07 | 5.04 | | | |
| pH 5.5 acetate | Doxorubicin·HCl assay | | | | | | | | | |
| | . mg/ml | 1.991 | 1.841 | 1.470 | 1.246 | 1.091 | | | | |
| | . % stability | 100.0 | 92.5 | 73.8 | 62.6 | 54.8 | | | | |
| | pH | 5.56 | 5.54 | 5.48 | 5.50 | 5.46 | | | | |

EP 1 283 044 A2

Table 3 - Accelerated (55°C) stability data of 2 mg/ml doxorubicin·HCl solutions in 0.9% saline at various pHs

| Buffers | Tests | Time (hours) | | | | | | | | | |
|---|---|---|---|---|---|---|---|---|---|---|---|
| | | 0 | 4 | 8 | 16 | 24 | 34 | 48 | 72 | 96 | 120 |
| pH 2.0 glycine- | Doxorubicin·HCl assay | | | | | | | | | | |
| | . mg/ml | 1.998 | | 1.857 | 1.580 | 1.397 | 1.231 | 0.931 | 0.701 | | |
| | . % stability | 100.0 | | 92.9 | 79.1 | 69.9 | 61.6 | 46.6 | 35.1 | | |
| | pH | 2.16 | | 2.16 | 2.18 | 2.16 | 2.22 | 2.20 | 2.19 | | |
| pH 2.5 glycine-HCl | Doxorubicin·HCl assay | | | | | | | | | | |
| | . mg/ml | 1.946 | | 1.875 | 1.670 | 1.602 | 1.360 | 1.132 | | | |
| | . % stability | 100.0 | | 96.3 | 85.8 | 82.3 | 70.3 | 58.1 | | | |
| | pH | 2.59 | | 2.59 | 2.59 | 2.58 | 2.62 | 2.62 | | | |
| pH 3.0 glycine-HCl | Doxorubicin·HCl assay | | | | | | | | | | |
| | . mg/ml | 1.994 | | | 1.818 | 1.771 | | 1.571 | 1.375 | 1.205 | 1.003 |
| | . % stability | 100.0 | | | 91.2 | 88.8 | | 78.8 | 69.0 | 60.4 | 50.3 |
| | pH | 3.06 | | | 3.07 | 3.07 | | 3.08 | 3.13 | 3.14 | 3.12 |
| pH 3.5 formate | Doxorubicin·HCl assay | | | | | | | | | | |
| | . mg/ml | 1.997 | | | 1.824 | 1.742 | | 1.543 | 1.323 | 1.176 | 0.919 |
| | . % stability | 100.0 | | | 91.4 | 87.2 | | 77.3 | 66.2 | 58.9 | 46.0 |
| | pH | 3.58 | | | 3.56 | 3.56 | | 3.66 | 3.61 | 3.64 | 3.63 |
| pH 4.0 acetate | Doxorubicin·HCl assay | | | | | | | | | | |
| | . mg/ml | 1.972 | 1.885 | 1.828 | 1.653 | 1.594 | | | | | |
| | . % stability | 100.0 | 95.6 | 92.7 | 83.8 | 80.8 | | | | | |
| | pH | 4.10 | 4.10 | 4.10 | 4.10 | 4.11 | | | | | |
| pH 5.0 acetate | Doxorubicin·HCl assay | | | | | | | | | | |
| | . mg/ml | 1.979 | | 1.732 | 1.469 | 1.442 | 1.278 | | | | |
| | . % stability | 100.0 | | 87.5 | 74.2 | 72.8 | 64.6 | | | | |
| | pH | 5.04 | | 5.06 | 5.04 | 5.05 | 5.05 | | | | |
| pH 5.5 acetate | Doxorubicin·HCl assay | | | | | | | | | | |
| | . mg/ml | 2.023 | | 1.807 | 1.508 | 1.330 | | | | | |
| | . % stability | 100.0 | | 91.3 | 76.5 | 65.7 | | | | | |
| | pH | 5.58 | | 5.56 | 5.55 | 5.53 | | | | | |

Table 4 -

| $K_{obs}$ values (1/days) for the degradation of doxorubicin.HCl 2 mg/ml solutions in sterile water at various pHs at 55°C | | | |
|---|---|---|---|
| Buffer | pH | $K_{obs} \times 10^3$ | 95% confidence limits |
| . Glycine-HCl (I = 0.05) | 2.0 | 309.5 | ± 12.7 |
| . Glycine-HCl (I = 0.05) | 2.5 | 138.3 | ± 0.6 |

Table 4 -   (continued)

| $K_{obs}$ values (1/days) for the degradation of doxorubicin.HCl 2 mg/ml solutions in sterile water at various pHs at 55°C | | | |
|---|---|---|---|
| Buffer | pH | $K_{obs}$ x $10^3$ | 95% confidence limits |
| . Glycine-HCl (I = 0.05) | 3.0 | 93.1 | ± 4.6 |
| . Formate (I = 0.05) | 3.5 | 96.7 | ± 4.4 |
| . Acetate (I = 0.05) | 4.0 | 269.8 | ± 18.7 |
| . Acetate (I = 0.05) | 5.0 | 322.6 | ± 19.2 |
| . Acetate (I = 0.05) | 5.5 | 415.4 | ± 45.7 |

Table 5 -

| $K_{obs}$ values (1/days) for the degradation of doxorubicin.HCl 2 mg/ml solutions in 5% dextrose at various pHs at 55°C | | | |
|---|---|---|---|
| Buffer | pH | $K_{obs}$ x $10^3$ | 95% confidence limits |
| . Glycine-HCl (I = 0.05) | 2.0 | 323.8 | ± 17.2 |
| . Glycine-HCl (I = 0.05) | 2.5 | 138.7 | ± 9.9 |
| . Glycine-HCl (I = 0.05) | 3.0 | 100.5 | ± 5.9 |
| . Formate (I = 0.05) | 3.5 | 132.0 | ± 20.7 |
| . Acetate (I = 0.05) | 4.0 | 209.7 | ± 12.7 |
| . Acetate (I = 0.05) | 5.0 | 273.1 | ± 27.7 |
| . Acetate (I = 0.05) | 5.5 | 453.7 | ± 59.2 |

Table 6 -

| $K_{obs}$ values (1/days) for the degradation of doxorubicin.HCl 2 mg/ml solutions in 0.9% saline at various pHs at 55°C | | | |
|---|---|---|---|
| Buffer | pH | $K_{obs}$ x $10^3$ | 95% confidence limis |
| . Glyclne-HCl (I = 0.05) | 2.0 | 362.4 | ± 19.4 |
| . Glycine-HCl (I = 0.05) | 2.5 | 276.5 | ± 30.2 |
| . Glycine-HCl (I = 0.05) | 3.0 | 133.2 | ± 8.0 |
| . Formate (I = 0.05) | 3.5 | 148.1 | ± 11.1 |
| . Acetate (I = 0.05) | 4.0 | 215.7 | ± 35.4 |
| . Acetate (I = 0.05) | 5.0 | 301.2 | ± 60.1 |
| . Acetate (I = 0.05) | 5.5 | 430.3 | ± 59.9 |

Example 2: Shelf-life (t 90%) forecast of doxorubicin.HCl 2 mg/ml sterile water solution adjusted to pH 3.0

Doxorubicin.HCl was dissolved at a concentration of 2 mg/ml in Water for Injections and adjusted to pH 3.0 with 0.5 N HCl. The solution was filtered through a 0.22 μm microporous membrane under nitrogen pressure. 5.0 ml aliquots were stored at:

    (a) 55°C for 21 days,
    (b) 45°C and 35°C for 28 days, and
    (c) 27°C for 90 days.

in glass vials of glass type I, 8ml top capacity vial, Teflon-faced chlorobutyl rubber bung, aluminium seal. At predetermined times the vials were analysed for doxorubicin.HCl assay and pH. The results are shown in Table 7.
The logarithm of the residual doxorubicin.HCl concentration versus time was plotted for each set of results. A linear relationship was revealed, indicating that the degradation of the drug followed pseudo-first order kinetics at constant pH and temperature. The observed rate constants ($K_{obs}$) for the degradation were calculated again by linear regression analysis of a plot of the natural logarithm of the residual concentration of doxorubicin.HCl ($|Dx|_t$) versus time as depicted by the equation previously reported:

$$\ln |Dx|_t = \ln |Dx|_o - K_{obs} \cdot t$$

The Arrhenius equation for the degradation process was calculated from the $K_{obs}$ obtained from the different temperatures taken in account for the testing (Table 8). Applying the equation, the rate constants for the pseudo-first order reactions at 4°C, 8°C, 15°C and 27°C were calculated, together with the expected shelf-life ($t_{90\%}$) at these temperatures. The $t_{90\%}$ forecasts in Table 8 show that a meaningful shelf life can be attributed to doxorubicin.HCl 2mg/ml pH 3.0 aqueous solution if the product is stored in a refrigerator between 2°C and 8°C.

Table 7 - Accelerated stability data of doxorubicin·HCl 2 mg/ml pH 3.0 solutions in sterile water at different times and temperatures

| Storage temperature | Tests | Time (days) | | | | | | | |
|---|---|---|---|---|---|---|---|---|---|
| | | 0 | 4 | 8 | 14 | 21 | 28 | 60 | 90 |
| 27°C | Doxorubicin·HCl assay | | | | | | | | |
| | . mg/ml | 1.992 | | 1.993 | 1.988 | 1.962 | 1.941 | 1.908 | 1.850 |
| | . % stability | 100.0 | | 100.1 | 99.8 | 98.5 | 97.4 | 95.8 | 92.9 |
| | pH | 3.00 | | 2.95 | 2.94 | 2.95 | 2.94 | 2.96 | 2.93 |
| 35°C | Doxorubicin·HCl assay | | | | | | | | |
| | . mg/ml | 1.992 | 1.985 | 1.952 | 1.889 | 1.876 | 1.808 | | |
| | . % stability | 100.0 | 99.6 | 98.0 | 94.8 | 94.2 | 90.8 | | |
| | pH | 3.00 | 2.96 | 2.98 | 2.93 | 2.92 | 2.92 | | |
| 45°C | Doxorubicin·HCl assay | | | | | | | | |
| | . mg/ml | 1.992 | 1.919 | 1.851 | 1.677 | 1.565 | 1.393 | | |
| | . % stability | 100.0 | 96.3 | 92.9 | 84.2 | 78.6 | 69.9 | | |
| | pH | 3.00 | 2.97 | 2.95 | 2.85 | 2.92 | 2.90 | | |
| 55°C | Doxorubicin·HCl assay | | | | | | | | |
| | . mg/ml | 1.992 | 1.760 | 1.493 | 1.036 | 0.730 | | | |
| | .% stability | 100.0 | 88.4 | 74.9 | 52.0 | 36.6 | | | |
| | pH | 3.00 | 2.94 | 2.90 | 2.80 | 2.82 | | | |

EP 1 283 044 A2

Table 8 -

| Arrhenius approach. Pseudo-first order rate constants, Arrhenius equation, calculated $t_{90\%}$ | | | |
|---|---|---|---|
| . PSEUDO-FIRST ORDER RATE CONSTANTS, OBSERVED VALUES ($K_{obs}$) | | | |
| Temperature | $K_{obs}$ x $10^3$ (1/days) | Correlation coefficient | |
| 27°C | 0.850 | 0.986 | |
| 35°C | 3.506 | 0.983 | |
| 45°C | 12.790 | 0.995 | |
| 55°C | 49.340 | 0.995 | |
| . ARRHENIUS EQUATION FROM 27°C, 35°C, 45°C AND 55°C RATE CONSTANTS | | | |
| $\ln K_{obs} = -\dfrac{14083}{T} + 39.95$ | | | |
| correlation coefficient = 0.9988 | | | |
| . PSEUDO-FIRST ORDER RATE CONSTANTS, CALCULATED VALUES (K) | | | |
| Temperature | K x $10^3$ (1/days) | $t_{90\%}$ (days) | 95% confidence limits |
| 4°C | 0.019 | 5,652 | 3,079 - 10,380 |
| 8°C | 0.038 | 2,745 | 1,603 - 4,697 |
| 15°C | 0.130 | 810 | 532 - 1,238 |
| 27°C | 0.918 | 115 | 89 - 147 |

Example 3: Long term stability of doxorubicin formulations having a pH of from 2.5 to 3.5

Batches tested, formulations tested and packaging used are shown in Tables 9 to 11 and 18 to 20. The formulations were prepared as follows.

Doxorubicin.HCl was dissolved in about 90 percent of the stated amount of water for injections or physiological saline, deaerated by nitrogen bubbling. Hydrochloric acid was added dropwise to adjust the pH of the solution to the values reported in Tables 9 and 18. Deaerated water for injections or physiological saline was then added in order to obtain a doxorubicin.HCl concentration of 2 mg/ml. The solutions were filtered through a 0.22 μm microporous membrane under nitrogen pressure. Volumes of 5 ml, 10 ml and 25 ml of the solutions were distributed into type I, colourless. glass vials having 8 or 10 ml, 14 ml and 39 ml top capacity, respectively. The vials were then closed with chlorobutyl teflon-faced rubber stoppers and sealed with aluminium caps. The formulations thus prepared were tested as regards appearance, clarity of solutions, pH, sterility (8°C, yearly), doxorubicin.HCl assay.

Test methods

For appearance and clarity: visual inspection
For pH: USP XXI
For sterility: USP XXI (membrane filtration)
For doxorubicin.HCl assay: HPLC ion-pair method and USP HPLC method (USP XXI)
Brief description of the HPLC ion-pair method for doxorubicin.HCl assay:

| | |
|---|---|
| Column filling : | reverse phase, Zorbax TMS |
| Mobile phase : | water: acetonitrile: methanol (54:29:17 v/v/v) containing 2 ml/l 85% phosphoric acid and 1 mg/ml sodium laurylsulfate (pairing agent) adjusted to pH 3.5 with 2N NaOH |
| Mobile phase flow rate | : 1.5 ml/min |
| Column temperature | : ambient (22°C $\pm$ 2°C) |
| Analytical wavelength | : 254 nm |
| System suitability parameters | : symmetry factor between 0.7 an 1.2; number of theoretical plates $\geq$ 2500; measurement reproducibility: variation coefficient < 1, n=6; resolution factor $\geq$ 12 |

The HPLC ion-pair method for doxorubicin.HCl assay is validated for accuracy, precision, linearity, sensitivity, specificity and stability-indicating nature.
The results obtained for:

. percent doxorubicin.HCl stability (ion-pair method) and
.. pH

referred to the vials stored in upright position are given in:

Table 12 storage at -20°C
Tables 13 and 21 storage at + 4°C
Tables 14 and 22 storage at + 8°C
Tables 15 and 23 storage at +15°C
Tables 16 and 24 storage at +27°C
Table 17 storage at 100 and 250 foot candles
Table 25 storage at 250 foot candles.

The doxorubicin.HCl assays given in these Tables are the mean of three independent determinations. Also, the stabilizing effect of various agents is shown in Table 26.

As far as the other parameters checked during stability:

- the clarity of the solution was unchanged at all the checks carried out at all the storage conditions applied
- the appearance of the solutions was: a) unchanged at all the checks carried out on samples stored at 4°C and 8°C, b) slightly darkened after: 9 months at 15°C, 3 months at 27°C, 3 months at 100 and 250 foot candles light
- the closure system was unchanged at all the checks carried out at all the storage conditions
- the sterility was maintained after 12 months at 8°C.

The results of the controls carried out on the vials stored in the inverted position did not differ significantly from those on the vials in the upright position.

The percent doxorubicin.HCl stability values obtained by the USP HPLC method did not differ significantly from those obtained by the HPLC ion-pair method given in Tables 12-17.

The obtained stability data indicate that the tested doxorubicin.HCl solutions having different pH values within the range from 2.5 to 3.5 reach the lower limit of acceptance (90% of nominal concentration) in about 9 and 2-3 months at 15°C and, respectively, 27°C, but prove stable up to 12 months at 4°C and 8°C, i.e. at the temperature usually adopted for the storage of the products under refrigeration. This is in contrast to the doxorubicin.HCl solutions obtained upon reconstitution of the commercial freeze-dried preparate, whose pHs vary between 4.5 and 6 and which show a much lower stability. Indeed, it is recommended that reconstituted solutions should be discarded after only 48 hours storage in a refrigerator.

Table 9 — Stability studies. Batches tested

| Batch characteristics    Batch No. | TF/23049 | TF/23077 | TF/23078 | TF/23117 | TF/23119 | H0001 | L0001 | M0001 |
|---|---|---|---|---|---|---|---|---|
| Doxorubicin · HCl per vial (mg) | 10 | 10 | 10 | 20 | 50 | 10 | 20 | 50 |
| pH | 3.06 | 2.81 | 3.50 | 2.97 | 3.08 | 3.15 | 3.05 | 3.20 |
| Formulation No. | FI6804/IL1 | FI6804/IL1 | FI6804/IL1 | FI6804/IL2 | FI6804/IL3 | FI6804/IL4 | FI6804/IL5 | FI6804/IL6 |
| Batch size No. of vials | 700 | 400 | 400 | 500 | 500 | 2,400 | 2,300 | 2,400 |

EP 1 283 044 A2

Table 10 - Stability studies.  Formulations tested.

| Composition per vial | Formulation number | | | | | |
|---|---|---|---|---|---|---|
| | F16804/1L1 | F16804/1L2 | F16804/1L3 | F16804/1L4 | F16804/1L5 | F16804/1L6 |
| Doxorubicin·HCl mg | 10 | 20 | 50 | 10 | 20 | 50 |
| Hydrochloric acid q.s. to pH | 2.8 - 3.5 | 2.8 - 3.5 | 2.8 - 3.5 | 2.8 - 3.5 | 2.8 - 3.5 | 2.8 - 3.5 |
| Water q.s. to ml | 5.0 | 10.0 | 25.0 | 5.0 | 10.0 | 25.0 |

EP 1 283 044 A2

Table 11 — Stability studies. Packaging used

| Packaging \ Batch No. | TF/23049 | TF/23077 | TF/23078 | TF/23117 | TF/23119 | K0001 | L0001 | M0001 |
|---|---|---|---|---|---|---|---|---|
| vial glass type | I | I | I | I | I | I | I | I |
| vial top capacity | 8 ml | 8 ml | 8 ml | 14 ml | 39 ml | 10 ml | 14 ml | 39 ml |
| stopper | chlorobutyl rubber, teflon-faced | chlorobutyl rubber, teflon-faced | chlorobutyl rubber, teflon-faced | chlorobutyl rubber, teflon-faced | chlorobutyl rubber, teflon-faced | chlorobutyl rubber, teflon-faced | chlorobutyl rubber, teflon-faced | chlorobutyl rubber teflon-faced |
| seal | aluminium | aluminium | aluminium | aluminium | aluminium | aluminium | aluminium | aluminium |

EP 1 283 044 A2

Table 12 -

| Doxorubicin·HCl 2 mg/ml solution. Stability data at -20°C (vials stored upright) acquired up to 3 months. | | | | |
|---|---|---|---|---|
| | | Time - Months | | |
| Batch Dosage | | 0 | 1 | 3 |
| H0001 10 mg | doxorubicin·HCl % stability | 100 | 99.9 | 99.6 |
| | pH | 3.15 | 3.12 | 2.98 |
| L0001 20 mg | doxorubicin·HCl % stability | 100 | 100.8 | 99.8 |
| | pH | 3.05 | 2.84 | 2.97 |
| M0001 50 mg | doxorubicin·HCl % stability | 100 | 100.7 | 101.0 |
| | pH | 3.20 | 2.96 | 2.99 |

EP 1 283 044 A2

Table 13 — Doxorubicin·HCl 2 mg/ml solution.
Stability data at 4°C (vials stored upright)
acquired up to 24 months

| Batch Dosage | Time Months | 0 | 1 | 3 | 6 | 9 | 12 | 18 | 24 |
|---|---|---|---|---|---|---|---|---|---|
| TF/23049 | * | 100 | 99.9 | 100.6 | 98.3 | 98.2 | 97.7 | 96.9 | 97.1 |
| 10 mg | ** | 3.06 | 3.10 | 3.09 | 3.10 | 3.05 | 2.97 | 3.07 | 3.00 |
| TF/23077 | * | 100 | 101.7 | 99.3 | 97.9 | 98.0 | 99.8 | 96.4 | |
| 10 mg | ** | 2.81 | 2.86 | 2.75 | 2.65 | 2.67 | 2.76 | 2.70 | |
| TF/23078 | * | 100 | 101.2 | 98.8 | 97.8 | 98.8 | 96.8 | 96.3 | |
| 10 mg | ** | 3.50 | 3.54 | 3.49 | 3.44 | 3.43 | 3.54 | 3.50 | |
| TF/23117 | * | 100 | 96.8 | 96.6 | 98.1 | 98.8 | 97.5 | 98.7 | |
| 20 mg | ** | 2.97 | 2.98 | 2.92 | 2.86 | 2.95 | 2.98 | 3.00 | |
| TF/23119 | * | 100 | 98.6 | 99.1 | 98.9 | 98.4 | 97.5 | 98.8 | |
| 50 mg | ** | 3.08 | 2.98 | 2.98 | 2.89 | 2.99 | 3.00 | 3.00 | |
| H0001 | * | 100 | | 97.6 | 99.2 | 99.6 | 100.4 | 98.6 | |
| 10 mg | ** | 3.15 | n.d. | 3.06 | 3.22 | 3.20 | 3.10 | 3.20 | |
| L0001 | * | 100 | | 98.8 | 98.4 | 99.2 | 99.4 | 99.9 | |
| 20 mg | ** | 3.05 | n.d. | 2.99 | 2.94 | 3.00 | 2.90 | 3.00 | |
| M0001 | * | 100 | | 99.7 | 99.7 | 100.3 | 100.6 | 100.4 | |
| 50 mg | ** | 3.20 | n.d. | 3.00 | 3.04 | 3.10 | 3.00 | 3.10 | |

\* doxorubicin·HCl % stability

\*\* pH

n.d. = not determined

18

Table 14 — Doxoribicin·HCl 2 mg/ml solution.
Stability data at 8°C (vials stored upright)
acquired up to 24 months.

| Batch Dosage | Time Months | 0 | 1 | 2 | 3 | 6 | 9 | 12 | 18 | 24 |
|---|---|---|---|---|---|---|---|---|---|---|
| TF/23049 | • | 100 | 99.7 | | 100.1 | 96.5 | 96.1 | 96.5 | 95.4 | 91.0 |
| 10 mg | •• | 3.06 | 3.07 | | 3.09 | 3.07 | 3.04 | 2.96 | 3.04 | 3.00 |
| TF/23077 | • | 100 | 102.1 | | 101.6 | 97.5 | 96.6 | 95.0 | 94.7 | |
| 10 mg | •• | 2.81 | 2.81 | | 2.74 | 2.65 | 2.67 | 2.75 | 2.70 | |
| TF/23078 | • | 100 | 98.3 | | 97.7 | 96.5 | 95.9 | 98.8 | 92.8 | |
| 10 mg | •• | 3.50 | 3.59 | | 3.47 | 3.27 | 3.43 | 3.51 | 3.40 | |
| TF/23117 | • | 100 | 95.7 | | 95.8 | 97.8 | 96.2 | 95.5 | 95.9 | |
| 20 mg | •• | 2.97 | 2.97 | | 2.92 | 2.85 | 2.96 | 2.98 | 3.00 | |
| TF/23119 | • | 100 | 97.6 | | 97.8 | 96.2 | 97.3 | 96.8 | 97.2 | |
| 50 mg | •• | 3.08 | 2.94 | | 2.94 | 2.87 | 2.99 | 3.00 | 3.00 | |
| H0001 | • | 100 | 98.2 | 99.4 | 96.4 | 96.7 | 98.0 | 98.9 | 96.7 | |
| 10 mg | •• | 3.15 | 3.12 | 3.16 | 3.05 | 3.23 | 3.20 | 3.10 | 3.20 | |
| L0001 | • | 100 | 100.6 | 99.1 | 98.1 | 98.3 | 98.1 | 99.5 | 99.7 | |
| 20 mg | •• | 3.05 | 2.84 | 2.83 | 2.97 | 2.94 | 3.00 | 2.90 | 3.00 | |
| M0001 | • | 100 | 100.3 | 100.6 | 98.7 | 99.0 | 98.4 | 99.1 | 97.7 | |
| 50 mg | •• | 3.20 | 2.96 | 2.97 | 3.01 | 3.03 | 3.10 | 3.00 | 3.10 | |

• doxorubicin·HCl % stability

•• pH

Table 15 — Doxorubicin·HCl 2 mg/ml solution.
Stability data at 15°C (vials stored upright)
acquired up to 12 months.

| Batch Dosage | Time Months | 0 | 0.5 | 1 | 2 | 3 | 6 | 9 | 12 |
|---|---|---|---|---|---|---|---|---|---|
| TF/23049 | * | 100 | 97.8 | 98.9 | | 97.1 | 92.7 | 92.9 | 90.2 |
| 10 mg | ** | 3.06 | 3.03 | 3.07 | | 3.10 | 3.08 | 3.02 | 2.95 |
| TF/23077 | * | 100 | 100.4 | 101.9 | | 98.8 | 94.6 | 92.7 | 91.1 |
| 10 mg | ** | 2.81 | 2.81 | 2.85 | | 2.71 | 2.63 | 2.67 | 2.74 |
| TF/23078 | * | 100 | 101.4 | 98.4 | | 95.3 | 94.6 | 91.9 | 90.7 |
| 10 mg | ** | 3.50 | 3.51 | 3.58 | | 3.47 | 3.38 | 3.41 | 3.47 |
| TF/23117 | * | 100 | 99.1 | 96.4 | | 95.2 | 94.6 | 90.7 | 88.0 |
| 20 mg | ** | 2.97 | 2.95 | 2.95 | | 2.90 | 2.81 | 2.95 | 3.00 |
| TF/23119 | * | 100 | 97.4 | 97.1 | | 95.9 | 92.7 | 90.6 | 87.4 |
| 50 mg | ** | 3.08 | 2.99 | 2.95 | | 2.91 | 2.87 | 2.98 | 3.00 |
| H0001 | * | 100 | | 97.9 | 97.1 | 94.8 | 94.6 | 95.5 | 91.8 |
| 10 mg | ** | 3.15 | | 3.12 | 3.16 | 3.06 | 3.23 | 3.10 | 3.00 |
| L0001 | * | 100 | | 100.5 | 98.7 | 96.3 | 95.5 | 96.3 | 93.2 |
| 20 mg | ** | 3.05 | | 2.85 | 2.87 | 2.98 | 2.96 | 3.00 | 3.00 |
| M0001 | * | 100 | | 99.4 | 100.3 | 97.2 | 95.6 | 95.6 | 93.7 |
| 50 mg | ** | 3.20 | | 2.96 | 2.94 | 3.01 | 3.04 | 3.00 | 3.00 |

* doxorubicin·HCl % stability
** pH

Table 16 — Doxorubicin·HCl 2 mg/ml solution.
Stability data at 27°C (vials stored upright)
acquired up to 6 months.

| Batch Dosage | Time Months | 0 | 0.5 | 1 | 2 | 3 | 6 |
|---|---|---|---|---|---|---|---|
| TF/23049 10 mg | * | 100 | 97.2 | 95.8 | | 87.9 | 73.6 |
| | ** | 3.06 | 2.98 | 3.07 | | 3.08 | 3.03 |
| TF/23077 10 mg | * | 100 | 98.5 | 96.2 | | 86.4 | 69.2 |
| | ** | 2.81 | 2.80 | 2.85 | | 2.71 | 2.64 |
| TF/23078 10 mg | * | 100 | 101.2 | 94.5 | | 80.5 | 71.1 |
| | ** | 3.50 | 3.51 | 3.58 | | 3.38 | 3.13 |
| TF/23117 20 mg | * | 100 | 97.4 | 93.2 | | 81.9 | 66.6 |
| | ** | 2.97 | 2.95 | 2.94 | | 2.88 | 2.77 |
| TF/23119 50 mg | * | 100 | 96.0 | 93.3 | | 85.3 | 66.8 |
| | ** | 3.08 | 2.97 | 2.97 | | 2.91 | 2.82 |
| H0001 10 mg | * | 100 | | 94.5 | 94.2 | 86.6 | |
| | ** | 3.15 | | 3.10 | 3.09 | 3.01 | |
| L0001 20 mg | * | 100 | | 97.2 | 94.3 | 89.3 | |
| | ** | 3.05 | | 2.84 | 2.85 | 2.96 | |
| M0001 50 mg | * | 100 | | 96.5 | 93.6 | 88.1 | |
| | ** | 3.20 | | 2.95 | 2.95 | 2.99 | |

\* doxorubicin·HCl % stability
\*\* pH

Table 17 -- Doxorubicin·HCl 2 mg/ml solution. Stability data at 100 and 250 f.c. (vials stored inverted) acquired up to 3 months.

| Batch Dosage | Time Months | 100 foot-candles | | | | 250 foot-candles | | | |
|---|---|---|---|---|---|---|---|---|---|
| | | 0 | 0.5 | 1 | 3 | 0.5 | 1 | 2 | 3 |
| TF/23049 | * | 100 | 96.3 | 95.9 | 81.3 | 95.9 | 94.8 | | |
| 10 mg | ** | 3.06 | 3.05 | 3.05 | 3.06 | 2.99 | 3.04 | | |
| TF/23077 | * | 100 | 98.3 | 98.1 | 87.7 | 97.3 | 94.5 | | |
| 10 mg | ** | 2.81 | 2.79 | 2.84 | 2.70 | 2.79 | 2.84 | | |
| TF/23078 | * | 100 | 99.6 | 96.4 | 88.0 | 97.8 | 89.7 | | |
| 10 mg | ** | 3.50 | 3.50 | 3.58 | 3.39 | 3.47 | 3.53 | | |
| TF/23117 | * | 100 | 96.8 | 96.7 | 91.7 | 98.1 | 94.6 | | |
| 20 mg | ** | 2.97 | 2.93 | 2.95 | 2.87 | 2.95 | 2.93 | | |
| TF/23119 | * | 100 | 96.9 | 96.7 | 89.6 | 96.4 | 95.0 | | |
| 50 mg | ** | 3.08 | 2.96 | 2.95 | 2.93 | 2.96 | 2.97 | | |
| H0001 | * | 100 | | | | | 95.2 | 93.7 | 87.8 |
| 10 mg | ** | 3.15 | | | | | 3.10 | 3.06 | 2.97 |
| L0001 | * | 100 | | | | | 96.5 | 93.0 | 86.5 |
| 20 mg | ** | 3.05 | | | | | 2.84 | 2.85 | 2.97 |
| M0001 | * | 100 | | | | | 97.8 | 91.5 | 85.3 |
| 50 mg | ** | 3.20 | | | | | 2.95 | 2.94 | 2.99 |

*   doxorubicin·HCl % stability
**  pH

Table 18 - Stability studies. Batches tested.

| Batch Characteristics | Batch No. | | |
|---|---|---|---|
| | P0001 | Q0001 | R0001 |
| Doxorubicin·HCl per vial (mg) | 10 | 20 | 50 |
| pH | 3.00 | 3.00 | 3.00 |
| Formulation No. | FI6804/IL7 | FI6804/IL8 | FI6804/IL9 |
| Batch size No.of vials | 2,400 | 2,200 | 2,500 |

EP 1 283 044 A2

Table 19 - Stability studies. Formulations tested.

| Composition per vial | Formulation number | | |
|---|---|---|---|
| | F16804/IL7 | F16804/IL8 | F16804/IL9 |
| Doxorubicin·HCl mg | 10 | 20 | 50 |
| Hydrochloric acid q.s. to pH | 2.8 - 3.5 | 2.8 - 3.5 | 2.8 - 3.5 |
| 0.9% sodium chloride injection q.s.to ml | 5.0 | 10.0 | 25.0 |

EP 1 283 044 A2

Table 20 - Stability studies. Packaging used.

| Packaging | Batch No. | | |
|---|---|---|---|
| | P0001 | Q0001 | R0001 |
| vial glass type | I | I | I |
| vial top capacity | 10 ml | 14 ml | 39 ml |
| stopper | chlorobutyl rubber, teflon-faced | chlorobutyl rubber, teflon-faced | chlorobutyl rubber, teflon-faced |
| seal | aluminum | aluminum | aluminum |

Table 21 -

| Doxorubicin·HCl 2 mg/ml solution in Saline for Injection at pH=3. Stability data at 4°C (vials stored upright) acquired up to 12 and 18 months. | | | | | | | |
|---|---|---|---|---|---|---|---|
| Batch Dosage | | Time - Months | | | | | |
| | | 0 | 3 | 6 | 9 | 12 | 18 |
| P0001 10 mg | doxorubicin·HCl % stability | 100 | 98.3 | 98.0 | 99.2 | 97.9 | |
| | pH | 3.00 | 2.93 | 2.98 | 2.90 | 2.90 | |

Table 21 -   (continued)

| Doxorubicin·HCl 2 mg/ml solution in Saline for Injection at pH=3. Stability data at 4°C (vials stored upright) acquired up to 12 and 18 months. | | | | | | | |
|---|---|---|---|---|---|---|---|
| Batch Dosage | | Time - Months | | | | | |
| | | 0 | 3 | 6 | 9 | 12 | 18 |
| Q0001 20 mg | doxorubicin·HCl % stability | 100 | 97.5 | 97.0 | 100.1 | 98.9 | |
| | pH | 3.01 | 3.06 | 3.03 | 3.00 | 3.00 | |
| R0001 50 mg | doxorubicin·HCl % stability | 100 | 99.8 | 100.7 | 101.2 | 101.7 | 100.9 |
| | pH | 3.02 | 3.08 | 3.15 | 3.14 | 3.10 | 3.10 |

Table 22 -

| Doxorubicin·HCl 2 mg/ml solution in Saline for Injection at pH=3. Stability data at 8°C (vials stored upright) acquired up to 12and 18 months. | | | | | | | | | |
|---|---|---|---|---|---|---|---|---|---|
| Batch Dosage | | Time - Months | | | | | | | |
| | | 0 | 1 | 2 | 3 | 6 | 9 | 12 | 18 |
| P0001 10 mg | doxorubicin·HCl % stability | 100 | 101.0 | 100.6 | 97.9 | 97.4 | 96.8 | 96.1 | |
| | pH | 3.00 | 2.93 | 2.89 | 2.91 | 3.00 | 2.90 | 3.00 | |
| Q0001 20 mg | doxorubicin·HCl % stability | 100 | 99.4 | 99.9 | 96.8 | 96.7 | 95.7 | 96.0 | |
| | pH | 3.01 | 3.02 | 3.01 | 3.05 | 3.02 | 3.00 | 3.00 | |
| R0001 50 mg | doxorubicin·HCl % stability | 100 | 99.8 | 99.8 | 98.4 | 98.5 | 99.5 | 100.9 | 99.7 |
| | pH | 3.02 | 3.02 | 3.09 | 3.08 | 3.13 | 3.13 | 3.10 | 3.10 |

Table 23 -

| Doxorubicin·HCl 2 mg/ml solution in Saline for Injection. at pH=3. Stability data at 15°C (vials stored upright) acquired up to 12 months | | | | | | | | |
|---|---|---|---|---|---|---|---|---|
| Batch Dosage | | Time - Months | | | | | | |
| | | 0 | 1 | 2 | 3 | 6 | 9 | 12 |
| P0001 10 mg | doxorubicin·HCl % stability | 100 | 100.6 | 99.9 | 95.9 | 94.0 | 89.1 | 89.7 |
| | pH | 3.00 | 2.93 | 2.89 | 2.90 | 2.99 | 2.90 | 2.90 |
| Q0001 20 mg | doxorubicin·HCl % stability | 100 | 98.6 | 97.8 | 95.1 | 96.4 | 89.8 | 88.3 |
| | pH | 3.01 | 3.01 | 3.01 | 3.04 | 3.01 | 3.00 | 3.00 |
| R0001 50 mg | doxorubicin·HCl % stability | 100 | 98.8 | 97.5 | 97.6 | 94.7 | 96.0 | 94.5 |
| | pH | 3.02 | 3.02 | 3.08 | 3.08 | 3.14 | 3.11 | 3.10 |

Table 24 -

| Doxorubicin·HCl 2 mg/mL solution in Saline for Injection at pH=3. Stability data at 27°C (vials stored upright) acquired up to 3 months. | | | | | |
|---|---|---|---|---|---|
| Batch Dosage | | Time - Months | | | |
| | | 0 | 1 | 2 | 3 |
| P0001 10 mg | doxorubicin·HCl % stability | 100 | 98.3 | 95.0 | 84.9 |
| | pH | 3.00 | 2.93 | 2.89 | 2.88 |
| Q0001 20 mg | doxorubicin·HCl % stability | 100 | 96.0 | 93.2 | 83.8 |
| | pH | 3.01 | 3.01 | 2.99 | 3.03 |
| R0001 50 mg | doxorubicin·HCl % stability | 100 | 95.6 | 92.2 | 88.7 |
| | pH | 3.02 | 3.02 | 3.06 | 3.05 |

Table 25 -

| Doxorubicin·HCl 2 mg/ml solution in Saline for Injection at pH=3. Stability data at R.T.+250 f.c. (vials stored upright) acquired up to 3 months. | | | | | |
|---|---|---|---|---|---|
| Batch Dosage | | Time - Months | | | |
| | | 0 | 1 | 2 | 3 |
| P0001 10 mg | doxorubicin·HCl | | | | |
| | % stability | 100 | 89.6 | 86.5 | 70.3 |
| | pH | 3.00 | 2.92 | 2.86 | 2.84 |
| Q0001 20 mg | doxorubicin·HCl | | | | |
| | % stability | 100 | 91.1 | 84.5 | 72.7 |
| | pH | 3.01 | 2.99 | 2.97 | 2.98 |
| R0001 50 mg | doxorubicin·HCl | | | | |
| | % stability | 100 | 96.0 | 91.4 | 86.6 |
| | pH | 3.02 | 3.01 | 3.04 | 3.02 |

Stabilizing effect of various agents.

Table 26 -

| Stability Data of Doxorubicin Solution, 2 mg/ml and a pH of 3.0 at 45°C | | | | |
|---|---|---|---|---|
| Stabilizing Agent and Its Concentration | % Initial | | | |
| | 1 Wk | 2 Wk | 4 Wk | 8 Wk |
| Water | 87.8 | 75.9 | 53.8 | 25.5 |
| 5% Dextrose | 91.1 | 82.3 | 65.6 | 38.8 |
| 5% Galactose | 91.5 | 86.1 | 64.3 | -- |
| 5% Fructose | 91.9 | 80.6 | 64.1 | -- |
| 4% α-L(-)-Fucose | 91.2 | 81.9 | 63.8 | -- |
| 4% α-D(+)-Fucose | 91.8 | 81.9 | 63.3 | -- |
| 1% Lactose | 91.3 | 81.7 | 64.5 | 34.8 |
| 4% Dextran, MW 9,000 | 90.5 | 81.5 | -- | -- |
| 4% Dextran, MW 506,000 | 92.0 | 84.0 | -- | -- |
| 4% α-Cyclodextrin | 91.7 | 84.3 | -- | -- |
| 4% β-Cyclodextrin | 92.1 | 84.1 | -- | -- |
| 4% γ-Cyclodextrin | 94.3 | 89.0 | -- | -- |
| 5% Mannitol | 90.7 | 81.4 | 65.8 | 41.1 |
| 5% Sorbitol | 91.4 | 83.0 | 67.2 | 42.5 |
| 0.5% Thioglycerol | 90.8 | 83.2 | 63.5 | -- |
| 5% Inositol | 91.7 | 84.9 | -- | -- |
| 5% Ethanol | 92.2 | 85.6 | -- | -- |
| 10% Glycerol | 92.2 | 83.4 | 65.5 | -- |
| Note: The same stabilizing effect may be seen for the above agents at lower concentrations, e.g., lower by up to 25-50 wt.%. | | | | |

Example 4: Shelf-life (t 90%) forecast of doxorubicin.HCl 2 mg/ml sterile water solution adjusted to pH 3.0 and containing 0.9% by weight sodium chloride

Doxorubicin.HCl was dissolved at a concentration of 2 mg/ml in Physiological Saline and adjusted to pH 3.0 with 0.5 N HCl. The solution was filtered through a 0.22 μm microporous membrane under nitrogen pressure. 5.0 ml aliquots were stored at:

(a) 60°C for 4 days,
(b) 55°C for 11 days,
(c) 45°C for 21 days, and
(d) 35°C for 28 days

in glass vials of glass type I, 8ml top capacity vial, Teflon-faced chlorobutyl rubber bung, aluminium seal. At predetermined times the vials were analysed for doxorubicin.HCl assay and pH. The results are shown in Table 27.

The logarithm of the residual doxorubicin.HCl concentration versus time was plotted for each set of results. A linear relationship was revealed, indicating that the degradation of the drug followed pseudo-first order kinetics at constant pH and temperature. The observed rate constants ($K_{obs}$) for the degradation were calculated again by linear regression analysis of a plot of the natural logarithm of the residual concentration of doxorubicin.HCl ($|Dx|_t$) versus time as depicted by the equation previously reported:

$$\ln |Dx|_t = \ln |Dx|_o - K_{obs} \cdot t$$

The Arrhenius equation for the degradation process was calculated from the $K_{obs}$ obtained from the different temperatures taken in account for the testing (Table 28). Applying the equation, the rate constants for the pseudo-first order reactions at 4°C, 8°C, 15°C and 27°C were calculated, together with the expected shelf-life ($t_{90\%}$) at these temperatures. The $t_{90\%}$ forecasts in Table 28 show that a meaningful shelf life can be attributed to doxorubicin.HCl 2mg/ml pH 3.0 aqueous solution in 0.9% sodium chloride if the product is stored in a refrigerator between 2°C and 8°C.

Table 27 - Accelerated stability data of doxorubicin·HCl 2 mg/ml in 0.9% sodium chloride at different times and temperatures

| Storage Conditions | Tests | Time (days) | | | | | | | | | |
|---|---|---|---|---|---|---|---|---|---|---|---|
| | | 0 | 1 | 2 | 3 | 4 | 8 | 11 | 14 | 21 | 28 |
| 35°C | Doxorubicin·HCl assay | | | | | | | | | | |
| | . mg/ml | 2.061 | | | | | 2.045 | | 1.946 | 1.932 | 1.852 |
| | . % stability | 100.0 | | | | | 99.2 | | 94.4 | 93.7 | 89.9 |
| | pH | 3.05 | | | | | 2.98 | | 2.92 | 2.92 | 2.98 |
| 45°C | Doxorubicin·HCl assay | | | | | | | | | | |
| | . mg/ml | 2.061 | | | | 1.996 | 1.724 | | 1.517 | 1.344 | |
| | . % stability | 100.0 | | | | 96.5 | 83.6 | | 73.6 | 65.2 | |
| | pH | 3.05 | | | | 2.98 | 2.97 | | 2.98 | 2.93 | |
| 55°C | Doxorubicin·HCl assay | | | | | | | | | | |
| | . mg/ml | 2.061 | | | | 1.450 | 1.066 | 0.900 | | | |
| | . % stability | 100.0 | | | | 70.4 | 51.7 | 43.7 | | | |
| | pH | 3.05 | | | | 2.90 | 2.97 | 2.95 | | | |
| 60°C | Doxorubicin·HCl assay | | | | | | | | | | |
| | . mg/ml | 2.061 | 1.742 | 1.481 | 1.290 | 1.050 | | | | | |
| | . % stability | 100.0 | 84.5 | 71.9 | 62.6 | 50.9 | | | | | |
| | pH | 3.05 | 2.97 | 2.96 | 2.98 | 2.96 | | | | | |

EP 1 283 044 A2

Table 28 -

| Doxorubicin.HCl 2 mg/ml pH 3.0 solution in 0.9% NaCl. Arrhenius approach. Pseudo-first order rate constants, Arrhenius equation, calculated $t_{90\%}$. | | |
|---|---|---|
| . PSEUDO-FIRST ORDER RATE CONSTANTS, OBSERVED VALUES ($K_{obs}$) | | |
| Temperature | $K_{obs}$ x $10^3$ (1/days) | Correlation coefficient |
| 35°C | 3.89 | 0.965 |
| 45°C | 21.61 | 0.987 |
| 55°C | 75.90 | 0.996 |
| 60°C | 164.9 | 0.998 |

| . ARRHENIUS EQUATION FROM 35°C, 45°C, 55°C, and 60°C RATE CONSTANTS |
|---|
| $\ln K_{obs} = -\dfrac{15100}{T} + 43.53$ |
| correlation coefficient = 0.9986 |

| . PSEUDO-FIRST ORDER RATE CONSTANTS, CALCULATED VALUES (K) | | | |
|---|---|---|---|
| Temperature | K x $10^3$ (1/days) | $t_{90\%}$ (days) | 95% confidence limits |
| 4°C | 0.017 | 6,166 | (1,670 - 22,756) |
| 8°C | 0.037 | 2,838 | ( 861 - 9,351) |
| 15°C | 0.137 | 768 | ( 281 - 2,105) |
| 27°C | 1.112 | 94 | ( 45 - 197 ) |

Example 5: Shelf-life (t 90%) forecast of doxorubicin.HCl 2 mg/ml sterile water solution adjusted to pH 3.0 and containing 5% by weight dextrose

Doxorubicin.HCl was dissolved at a concentration of 2 mg/ml in Water for Injections containing 5% by weight dextrose and adjusted to pH 3.0 with 0.5 N HCl. The solution was filtered through a 0.22 μm microporous membrane under nitrogen pressure. 5.0 ml aliquots were stored at:

    (a) 60°C for 8 days,
    (b) 55°C for 28 days,
    (c) 45°C for 28 days, and
    (d) 35°C for 28 days

in glass vials of glass type I, 8ml top capacity vial, Teflon-faced chlorobutyl rubber bung, aluminium seal. At predetermined times the vials were analysed for doxorubicin.HCl assay and pH. The results are shown in Table 29.

The logarithm of the residual doxorubicin.HCl concentration versus time was plotted for each set of results. A linear relationship was revealed, indicating that the degradation of the drug followed pseudo-first order kinetics at constant pH and temperature. The observed rate constants ($K_{obs}$) for the degradation were calculated again by linear regression analysis of a plot of the natural logarithm of the residual concentration of doxorubicin.HCl ( $|Dx|_t$) versus time as depicted by the equation previously reported:

$$\ln |Dx|_t = \ln |Dx|_o - K_{obs}. \, t$$

The Arrhenius equation for the degradation process was calculated from the $K_{obs}$ obtained from the different temperatures taken in account for the testing (Table 30). Applying the equation, the rate constants for the pseudo-first order reactions at 4°C, 8°C, 15°C and 27°C were calculated, together with the expected shelf-life ($t_{90\%}$) at these temperatures. The $t_{90\%}$ forecasts in Table 30 show that a meaningful shelf life can be attributed to doxorubicin.HCl 2mg/ml pH 3.0 aqueous solution containing 5% dextrose if the product is stored in a refrigerator between 2°C and 8°C.

EP 1 283 044 A2

Table 29 - Accelerated stability data of doxorubicin·HCl 2 mg/ml pH 3.0 solution in 5% dextrose at different times and temperatures

| Storage Conditions | Tests | 0 | 2 | 4 | 6 | 8 | 11 | 14 | 17 | 21 | 28 |
|---|---|---|---|---|---|---|---|---|---|---|---|
| | | | | | | Time (days) | | | | | |
| 35°C | Doxorubicin·HCl assay | | | | | | | | | | |
| | . mg/ml | 2.114 | | 2.044 | | 2.034 | | 2.015 | | 1.934 | 1.859 |
| | . % stability | 100.0 | | 96.7 | | 96.2 | | 53.3 | | 91.5 | 87.9 |
| | pH | 3.02 | | 2.98 | | 2.94 | | 2.95 | | 2.90 | 2.94 |
| 45°C | Doxorubicin·HCl assay | | | | | | | | | | |
| | . mg/ml | 2.114 | | 1.940 | | 1.870 | | 1.684 | | 1.510 | 1.410 |
| | . % stability | 100.0 | | 91.8 | | 88.5 | | 79.7 | | 71.5 | 66.7 |
| | pH | 3.02 | | 2.97 | | 2.98 | | 2.95 | | 2.96 | 2.96 |
| 55°C | Doxorubicin·HCl assay | | | | | | | | | | |
| | . mg/ml | 2.114 | | 1.718 | | 1.415 | | 1.112 | | 0.957 | 0.796 |
| | . % stability | 100.0 | | 81.3 | | 66.9 | | 52.6 | | 45.3 | 37.7 |
| | pH | 3.02 | | 2.95 | | 2.92 | | 2.99 | | 2.91 | 2.95 |
| 60°C | Doxorubicin·HCl assay | | | | | | | | | | |
| | . mg/ml | 2.114 | 1.752 | 1.393 | 1.176 | 0.925 | | | | | |
| | . % stability | 100.0 | 82.9 | 65.9 | 55.7 | 43.8 | | | | | |
| | pH | 3.02 | 2.96 | 2.98 | 2.96 | 2.97 | | | | | |

Table 30 -

| Doxorubicin.HCl 2 mg/ml pH 3.0 solution in 5% dextrose. Arrhenius approach. Pseudo-first order rate constants, Arrhenius equation, calculated $t_{90\%}$. | | | |
|---|---|---|---|
| . PSEUDO-FIRST ORDER RATE CONSTANTS, OBSERVED VALUES ($K_{obs}$) | | | |
| Temperature | $K_{obs}$ x $10^3$ (1/days) | Correlation coefficeint | |
| 35°C | 4.190 | 0.990 | |
| 45°C | 14.55 | 0.995 | |
| 55°C | 58.11 | 0.998 | |
| 60°C | 102.6 | 0.999 | |
| . ARRHENIUS EQUATION FROM 35°C, 45°C, 55°C, AND 60°C RATE CONSTANTS | | | |
| $\ln K_{obs} = -\dfrac{13266}{T} + 37.56$ <br><br> correlation coefficient = 0.9993 | | | |
| . PSEUDO-FIRST ORDER RATE CONSTANTS, CALCULATED VALUES (K) | | | |
| Temperature | K x $10^3$(1/days) | $t_{90\%}$ (days) | 95% confidence limits |
| 4°C | 0.0326 | 3,218 | (1,463 - 7,082) |
| 8°C | 0.0645 | 1,628 | ( 792 - 3,344) |
| 15°C | 0.203 | 516 | ( 281 - 949 ) |
| 27°C | 1.283 | 82 | ( 53 - 128 ) |

Example 6: Long term stability data for doxorubicin.HCl solutions at pH 3.7

A solution of doxorubicin.HCl (2 mg/ml) in sterile water solution and containing 0.9% by weight sodium chloride (batch TF/23256, 10 mg) was prepared. The solution was adjusted to pH 3.7 by 0.1 N hydrochloric acid. The stability data for the solution at different storage temperatures are shown in Table 31 below.

TABLE 31

| Storage temperature | Tests | Time (months) | | |
|---|---|---|---|---|
| | | 0 | 1 | 3 |
| 8°C | Doxorubicin.HCl assay (mg/ml) | 2.064 | 2.054 | 2.063 |
| | % stability | 100.0 | 99.3 | 100.0 |
| | pH | 3.7 | 3.7 | 3.6 |
| 15°C | Doxorubicin.HCl assay (mg/ml) | 2.064 | 2.073 | 2.029 |
| | % stability | 100.0 | 100.5 | 98.4 |
| | pH | 3.7 | 3.7 | 3.6 |
| 27°C | Doxorubicin.HCl assay (mg/ml) | 2.064 | 1.888 | 1.545 |
| | % stability | 100.0 | 91.5 | 74.9 |
| | pH | 3.7 | 3.6 | 3.3 |

Example 17: Stability of 4'-epi-doxorubicin (i.e.epirubicin) solutions

Solutions of epirubicin were prepared in the same fashion as the corresponding doxorubicin solutions hereinbefore. They were then tested for stability in the same way. The results are presented in Tables 32 to 61 below and illustrated in Figures 4 to 6.

Table IV - Accelerated (55°C) stability data of 2 mg/ml epirubicin.HCl solutions in sterile water at various pHs

| Buffers | Tests | 0 | 16 | 24 | 48 | 72 | 96 | 120 |
|---|---|---|---|---|---|---|---|---|
| | | | | | Time (hours) | | | |
| pH 2.0 glycine-HCl | Epirubicin.HCl assay | | | | | | | |
| | • mg/ml | 2.007 | 1.874 | 1.801 | 1.725 | 1.697 | 1.478 | 1.330 |
| | • % initial | 100.0 | 93.4 | 89.7 | 85.9 | 83.7 | 73.7 | 66.3 |
| | pH | 2.0 | 2.0 | 2.0 | 2.0 | 2.0 | 2.0 | 2.0 |
| pH 2.5 glycine-HCl | Epirubicin.HCl assay | | | | | | | |
| | • mg/ml | 2.033 | 1.972 | 1.923 | 1.823 | 1.777 | 1.622 | 1.572 |
| | • % initial | 100.0 | 99.9 | 94.6 | 89.7 | 87.4 | 79.8 | 77.3 |
| | pH | 2.5 | 2.4 | 2.5 | 2.5 | 2.4 | 2.4 | 2.4 |
| pH 3.0 glycine-HCl | Epirubicin.HCl assay | | | | | | | |
| | • mg/ml | 2.055 | 2.008 | 1.917 | 1.764 | 1.709 | 1.571 | 1.494 |
| | • % initial | 100.0 | 97.7 | 93.3 | 85.8 | 83.2 | 76.4 | 72.7 |
| | pH | 2.9 | 2.9 | 2.9 | 2.8 | 2.9 | 2.9 | 2.9 |
| pH 3.5 formate | Epirubicin.HCl assay | | | | | | | |
| | • mg/ml | 2.047 | 1.829 | 1.764 | 1.604 | 1.505 | 1.192 | 1.101 |
| | • % initial | 100.0 | 89.4 | 86.2 | 78.3 | 73.5 | 58.2 | 53.8 |
| | pH | 3.4 | 3.4 | 3.4 | 3.4 | 3.3 | 3.3 | 3.3 |
| pH 4.0 acetate | Epirubicin.HCl assay | | | | | | | |
| | • mg/ml | 2.011 | 1.848 | 1.442 | 1.301 | 0.964 | n.d. | 0.573 |
| | • % initial | 100.0 | 91.9 | 71.7 | 64.7 | 47.9 | n.d. | 28.5 |
| | pH | 4.0 | 4.0 | 4.0 | 4.0 | 4.0 | | 3.9 |
| pH 5.0 acetate | Epirubicin.HCl asssay | | | | | | | |
| | • mg/ml | 2.064 | 1.868 | 1.541 | 1.263 | 0.896 | | |
| | • % initial | 100.0 | 90.5 | 74.6 | 61.2 | 43.4 | n.d. | n.d. |
| | pH | 5.0 | 5.0 | 5.0 | 5.0 | 5.0 | | |
| pH 5.5 acetate | Epirubicin.HCl assay | | | | | | | |
| | • mg/ml | 2.056 | 1.794 | 1.426 | | | | |
| | • % initial | 100.0 | 87.3 | 69.4 | n.d. | n.d. | n.d. | n.d. |
| | pH | 5.5 | 5.5 | 5.4 | | | | |

n.d. = not determined

34

Table 33—Accelerated (55°C) stability data of 2 mg/ml epirubicin.HCl solutions in 0.9% Sodium Chloride Injection at various pHs

| Buffers | Tests | 0 | 4 | 8 | 16 | 24 | 36 | 48 | 72 | 96 | 120 | 144 |
|---|---|---|---|---|---|---|---|---|---|---|---|---|
| pH 1.5 HCl-KCl | Epirubicin.HCl assay | | | | | | | | | | | |
| | • mg/ml | 2.085 | | | 1.675 | 1.372 | 1.246 | 1.062 | | | | |
| | • % initial | 100.0 | n.d. | n.d. | 80.3 | 65.8 | 59.8 | 50.9 | n.d. | n.d. | n.d. | n.d. |
| | pH | 1.6 | | | 1.5 | 1.5 | 1.6 | 1.6 | | | | |
| pH 2.0 glycine-HCl | Epirubicin.HCl assay | | | | | | | | | | | |
| | • mg/ml | 2.077 | | 2.065 | 1.994 | 1.940 | 1.657 | 1.567 | 1.282 | 1.121 | 0.928 | |
| | • % initial | 100.0 | n.d. | 99.4 | 96.0 | 93.0 | 79.8 | 76.4 | 61.8 | 54.0 | 44.7 | n.d. |
| | pH | 2.0 | | 2.0 | 1.9 | 1.9 | 2.0 | 2.0 | 2.0 | 2.0 | 1.9 | |
| pH 2.5 glycine-HCl | Epirubicin.HCl assay | | | | | | | | | | | |
| | • mg/ml | 2.077 | | 2.066 | 1.987 | | | 1.781 | 1.665 | 1.449 | 1.285 | 1.175 |
| | • % initial | 100.0 | n.d. | 99.5 | 95.7 | n.d. | n.d. | 85.7 | 80.2 | 69.8 | 61.9 | 56.4 |
| | pH | 2.4 | | 2.4 | 2.4 | | | 2.4 | 2.4 | 2.4 | 2.4 | 2.4 |
| pH 3.0 glycine-HCl | Epirubicin.HCl assay | | | | | | | | | | | |
| | • mg/ml | 2.058 | | | 1.951 | 1.934 | 1.869 | 1.784 | 1.668 | 1.483 | 1.349 | 1.253 |
| | • % initial | 100.0 | n.d. | n.d. | 94.8 | 94.0 | 90.8 | 86.7 | 81.0 | 72.1 | 65.5 | 60.9 |
| | pH | 3.0 | | | 2.9 | 2.9 | 2.9 | 2.9 | 2.9 | 2.9 | 2.9 | 2.9 |
| pH 3.5 formate | Epirubicin.HCl assay | | | | | | | | | | | |
| | • mg/ml | 2.047 | | | 1.891 | | 1.711 | 1.662 | 1.360 | 1.265 | | |
| | • % initial | 100.0 | n.d. | n.d. | 92.4 | n.d. | 83.6 | 81.2 | 70.5 | 61.8 | n.d. | n.d. |
| | pH | 3.3 | | | 3.2 | | 3.3 | 3.3 | 3.2 | 3.2 | | |
| pH 4.0 acetate | Epirubicin.HCl assay | | | | | | | | | | | |
| | • mg/ml | 2.082 | 2.073 | | 1.892 | | 1.699 | 1.606 | 1.454 | | | |
| | • % initial | 100.0 | 99.3 | n.d. | 90.6 | n.d. | 81.3 | 76.9 | 69.4 | n.d. | n.d. | n.d. |
| | pH | 4.0 | 3.9 | | 3.9 | | 3.9 | 3.9 | 3.9 | | | |
| pH 5.0 acetate | Epirubicin.HCl assay | | | | | | | | | | | |
| | • mg/ml | 2.114 | 2.049 | 1.954 | 1.782 | 1.535 | | | | | | |
| | • % initial | 100.0 | 97.0 | 92.4 | 84.3 | 72.6 | n.d. | n.d. | n.d. | n.d. | n.d. | n.d. |
| | pH | 5.0 | 5.0 | 5.0 | 5.0 | 4.9 | | | | | | |

n.d. = not determined

Table 34 - Accelerated (55°C) stability data of 2 mg/ml epirubicin.HCl solutions in 5% Dextrose at various pHs

| Buffers | Tests | Time (hours) | | | | | | | | | | |
|---|---|---|---|---|---|---|---|---|---|---|---|---|
| | | 0 | 4 | 8 | 16 | 24 | 36 | 48 | 72 | 96 | 120 | 144 |
| pH 1.5 HCl-KCl | Epirubicin.HCl assay • mg/ml • % initial pH | 2.032 100.0 1.4 | n.d. | 1.922 94.6 1.4 | 1.782 87.7 1.4 | 1.649 81.1 1.4 | 1.418 69.8 1.4 | n.d. | 0.982 48.3 1.4 | 0.625 30.7 1.4 | n.d. | n.d. |
| pH 2.0 glycine-HCl | Epirubicin.HCl assay • mg/ml • % initial pH | 2.033 100.0 1.9 | n.d. | n.d. | 1.966 96.6 1.9 | 1.959 96.4 1.9 | 1.822 89.6 1.9 | 1.809 89.0 1.9 | 1.574 77.4 1.9 | 1.380 67.9 1.9 | 1.252 61.6 1.9 | 1.096 53.8 1.9 |
| pH 2.5 glycine-HCl | Epirubicin.HCl assay • mg/ml • % initial pH | 2.105 100.0 2.4 | n.d. | n.d. | 1.921 91.3 2.3 | 1.909 90.7 2.3 | 1.815 86.2 2.3 | 1.819 86.4 2.3 | 1.624 77.1 2.3 | 1.521 72.3 2.3 | n.d. | 1.264 60.0 2.3 |
| pH 3.0 glycine-HCl | Epirubicin.HCl assay • mg/ml • % initial pH | 2.029 100.0 2.9 | n.d. | 1.990 98.1 2.8 | 1.914 94.3 2.9 | 1.949 96.1 2.8 | 1.866 92.0 2.8 | 1.743 85.9 2.8 | n.d. | 1.562 77.0 2.8 | 1.442 71.1 2.8 | 1.318 65.0 2.8 |
| pH 3.5 formate | Epirubicin.HCl assay • mg/ml • % initial pH | 2.024 100.0 3.3 | n.d. | 1.949 96.3 3.3 | 1.938 95.7 3.3 | 1.851 91.4 3.3 | 1.807 89.3 3.3 | n.d. | 1.613 79.7 3.3 | 1.356 67.0 3.2 | n.d. | n.d. |
| pH 4.0 acetate | Epirubicin.HCl assay • mg/ml • % initial pH | 2.056 100.0 4.0 | 1.964 95.5 3.9 | 1.937 94.2 3.9 | n.d. | 1.787 86.9 4.0 | n.d. | 1.501 73.0 4.0 | n.d. | n.d. | n.d. | n.d. |
| pH 5.0 acetate | Epirubicin.HCl assay • mg/ml • % initial pH | 2.026 100.0 5.1 | 1.935 95.5 5.0 | 1.840 90.8 5.0 | 1.734 84.3 5.0 | 1.608 79.4 5.0 | 1.279 62.2 5.0 | n.d. | n.d. | n.d. | n.d. | n.d. |

n.d. = not determined

Table 35 -

| $K_{obs}$ values (1/days) for the degradation of epirubicin.HCl 2 mg/ml solutions in Water for Injection at various pHs at 55°C | | | |
|---|---|---|---|
| Buffer | pH | $K_{obs}$ x $10^3$ | 95% confidence limits |
| . Glycine-HCl (I = 0.05) | 2.0 | 79.0 | ± 6.9 |
| . Glycine-HCl (I = 0.05) | 2.5 | 55.1 | ± 4.0 |
| . Glycine-HCl (I = 0.05) | 3.0 | 66.8 | ± 5.4 |
| . Fermate (I = 0.05) | 3.5 | 125.4 | ± 17.1 |
| . Acetate (I = 0.05) | 4.0 | 376.7 | ± 60.6 |
| . Acetate (I = 0.05) | 5.0 | 430.4 | ± 42.8 |

Table 36 -

| K$_{obs}$ values (1/days) for the degradation of epirubitin.HCl 2 mg/ml solutions in 0.9% Sodium Chloride Injection at various pHs at 55°C | | | |
|---|---|---|---|
| Buffer | pH | K$_{obs}$ x 10$^3$ | 95% confidence limits |
| . HCl-KCl (I = 0.05) | 1.5 | 343.2 | ± 51.1 |
| . Glycine-HCl (I = 0.05) | 2.0 | 168.9 | ± 10.2 |
| . Glycine-HCl (I = 0.05) | 2.5 | 97.3 | ± 6.4 |
| . Glycine-HCl (I = 0.05) | 3.0 | 84.8 | ± 6.5 |
| . Formate (I = 0.05) | 3.5 | 124.5 | ± 14.0 |
| . Acetate (I = 0.05) | 4.0 | 124.8 | ± 13.6 |
| . Acetate (I= 0.05) | 5.0 | 318.1 | ± 52.1 |

Table 37 -

| K$_{obs}$ values (1/days) for the degradation of epirubitin.HCl 2 mg/ml solutions in 5% Dextrose solution at various pHs at 55°C | | | |
|---|---|---|---|
| Buffer | pH | K$_{obs}$ x 10$^3$ | 95% confidence limits |
| . HCl-KCl (I = 0.05) | 1.5 | 288.1 | ± 23.1 |
| . Glycine-HCl (I = 0.05) | 2.0 | 106.9 | ± 7.7 |
| . Glytine-HCl (I = 0.05) | 2.5 | 81.1 | ± 6.6 |
| . Glytine-HCl (I = 0.05) | 3.0 | 70.9 | ± 4.8 |
| . Formate (I = 0.05) | 3.5 | 89.1 | ± 9.3 |
| . Acetate (I = 0.05) | 4.0 | 150.6 | ± 17.0 |
| . Acetate (I = 0.05) | 5.0 | 225.7 | ± 12.9 |

Table 38 – Stability studies. Batches tested in 0.9% Sodium Chloride Injection

| Batch characteristics | Batch No. TF/23274 | TF/23275 | 6001LA |
|---|---|---|---|
| Epirubicin.HCl per vial (mg) | 10 | 10 | 10 |
| pH | 2.9 | 3.5 | 3.0 |
| Formulation No. | FI7701/IL2 | FI7701/IL2 | FI7701/IL2 |
| Batch size No of vials | 584 | 586 | 595 |
| Scale of manufacture * | Lab | Lab | S-i |
| Epirubicin.HCl batch No. | 6016G669 | 6016G669 | 6016G669 |

| Batch characteristics | Batch No. 6001LB | 6001LC |
|---|---|---|
| Epirubicin.HCl per vial (mg) | 20 | 50 |
| pH | 3.1 | 2.7 |
| Formulation No. | FI7701/IL3 | FI7701/IL4 |
| Batch size No of vials | 500 | 760 |
| Scale of manufacture * | S-i | S-i |
| Epirubicin.HCl batch No. | 6017G703 | 6017G703 |

*
Lab = Laboratory
S-i = Semi-industrial

Table 39 - Stability studies. Batches tested in 5% dextrose solution

| Batch No.<br>Batch<br>characteristics | TF/23270 | TF/23273 | 6001MA |
|---|---|---|---|
| Epirubicin.HCl<br>per vial (mg) | 10 | 10 | 10 |
| pH | 2.7 | 3.5 | 2.9 |
| Formulation No. | FI7701/IL5 | FI7701/IL5 | FI7701/IL5 |
| Batch size |  |  |  |
| No of vials | 564 | 573 | 553 |
| Scale of<br>manufacture * | Lab | Lab | S-i |
| Epirubicin.HCl batch<br>No. | 6016G669 | 6016G669 | 6016G669 |

| Batch No.<br>Batch<br>characteristics | 6001MB | 6001MC |
|---|---|---|
| Epirubicin.HCl<br>per vial (mg) | 20 | 50 |
| pH | 3.1 | 2.6 |
| Formulation No. | FI7701/IL6 | FI7701/IL7 |
| Batch size |  |  |
| No of vials | 500 | 751 |
| Scale of<br>manufacture * | S-i | S-i |
| Epirubicin.HCl batch<br>No. | 6017G703 | 6017G703 |

* Lab = Laboratory
S-i = Semi-industrial

Table 40 - Stability studies. Batch tested in Water for

Injection

| Batch No. | TF/23176 |
|---|---|
| Batch characteristics | |
| Epirubicin.HCl per vial (mg) | 10 |
| pH | 3.0 |
| Formulation No. | F17701/IL1 |
| Batch size | |
| No of vials | 430 |
| Scale of manufacture | Laboratory |
| Epirubicin.HCl batch No. | 5009D646 |

Table 41- Stability studies. Packaging used for epirubicin.HCl ready-to-use solution in 0.9% Sodium Chloride Injection

| Batch No. Packaging | TF/23274 | TF/23273 | 6001LA | 6001LB | 6001LC |
|---|---|---|---|---|---|
| vial glass type | 1 | 1 | 1 | 1 | 1 |
| vial top capacity | 10 ml | 10 ml | 10 ml | 14 ml | 39 ml |
| stopper | chlorobutyl rubber, teflon-faced | chlorobutyl rubber, teflon-faced | chlorobutyl rubber, teflon-faced | chlorobutyl rubber, teflon-faced | chlorobutyl rubber, teflon-faced |
| seal | aluminium | aluminium | aluminium | aluminium | aluminium |

Table 42 - Stability studies. Packaging used for epirubicin.HCl ready-to-use solution in 5% Dextrose

| Batch No. Packaging | TF/23270 | TF/23273 | 6001MA | 6001MB | 6001MC |
|---|---|---|---|---|---|
| vial glass type | 1 | 1 | 1 | 1 | 1 |
| vial top capacity | 10 ml | 10 ml | 10 ml | 14 ml | 39 ml |
| stopper | chlorobutyl rubber, teflon-faced | chlorobutyl rubber, teflon-faced | chlorobutyl rubber, teflon-faced | chlorobutyl rubber, teflon-faced | chlorobutyl rubber, teflon-faced |
| seal | aluminium | aluminium | aluminium | aluminium | aluminium |

EP 1 283 044 A2

Table 43 – Stability studies – Packaging used for epirubicin.HCl ready-to-use in Water for Injection

| Batch No. | TF/23176 |
|---|---|
| Packaging | |
| vial glass type | I |
| vial top capacity | 10 ml |
| stopper | chlorobutyl rubber, teflon-faced |
| seal | aluminium |

Table 44 -

| Stability data of epirubicin.HCl ready-to-use solution in 0.9% Sodium Chloride Injection, 4°C | | | |
|---|---|---|---|
| **Vials stored upright** | | | |
| Batch Dosage | | INITIAL CONTROL | 6 months |
| TF/23274 10 mg | . | 2.218 | |
| | .. | 100.0 | n.d. |
| | ... | 2.7 | |
| | .... | 2.9 | |

. epirubicin.HCl assay (mg/ml)

.. epirubicin.HCl % initial

... related substances %

.... pH n.d. = not determined

Table 44 -   (continued)

| Stability data of epirubicin.HCl ready-to-use solution in 0.9% Sodium Chloride Injection, 4°C | | | |
|---|---|---|---|
| **Vials stored upright** | | | |
| Batch Dosage | | INITIAL CONTROL | 6 months |
| TF/23275 10 mg | . | 2.223 | n.d. |
| | .. | 100.0 | |
| | ... | 2.6 | |
| | .... | 3.5 | |
| 6001LA 10 mg | . | 2.155 | 2.266 |
| | .. | 100.0 | 105.2 |
| | ... | 1.9 | 3.3 |
| | .... | 3.0 | 3.0 |
| 6001LB 20 mg | . | 1.961 | 2.013 |
| | .. | 100.0 | 102.6 |
| | ... | 1.2 | 3.3 |
| | .... | 3.1 | 3.1 |
| 6001LC 50 mg | . | 2.072 | 2.086 |
| | .. | 100.0 | 100.7 |
| | ... | 1.4 | 3.0 |
| | .... | 2.7 | 2.7 |

. epirubicin.HCl assay (mg/ml)

.. epirubicin.HCl % initial

... related substances %

.... pH n.d. = not determined

Table 45 -

| Stability data of epirubicin.HCl ready-to-use solution in 0.9% Sodium Chloride Injection, 8°C | | | | | |
|---|---|---|---|---|---|
| **Vials stored upright** | | | | | |
| Batch Dosage | | INITIAL CONTROL | 1 months | 3 months | 6 months |
| TF/23274 10 mg | . | 2.218 | n.d. | n.d. | n.d. |
| | .. | 100.0 | | | |
| | ... | 2.7 | | | |
| | .... | 2.9 | | | |
| TF/23275 10 mg | . | 2.223 | n.d. | n.d. | n.d. |
| | .. | 100.0 | | | |
| | ... | 2.8 | | | |
| | .... | 3.5 | | | |

. epirubicin.HCl assay (mg/ml)

.. epirubicin.HCl % initial

... related substances %

.... pH n.d. = not determined

Table 45 -   (continued)

| Stability data of epirubicin.HCl ready-to-use solution in 0.9% Sodium Chloride Injection, 8°C | | | | | |
|---|---|---|---|---|---|
| **Vials stored upright** | | | | | |
| Batch Dosage | | INITIAL CONTROL | 1 months | 3 months | 6 months |
| 6001LA 10 mg | . | 2.155 | 2.165 | 2.143 | 2.182 |
| | .. | 100.0 | 100.5 | 99.4 | 101.3 |
| | ... | 1.9 | 3.8 | 2.9 | 3.7 |
| | .... | 3.0 | 3.0 | 3.0 | 3.0 |
| 6001LB 20 mg | . | 1.961 | 1.983 | 1.949 | |
| | .. | 100.0 | 101.1 | 99.4 | n.d. |
| | ... | 1.2 | 2.6 | 3.0 | |
| | .... | 3.1 | 3.1 | 3.0 | |
| 6001LC 50 mg | . | 2.072 | 2.095 | 2.116 | 2.012 |
| | .. | 100.0 | 101.1 | 102.1 | 97.1 |
| | ... | 1.4 | 2.4 | 3.2 | 3.7 |
| | .... | 2.7 | 2.7 | 2.7 | 2.7 |

. epirubicin.HCl assay (mg/ml)

.. epirubicin.HCl % initial

... related substances %

.... pH n.d. = not determined

Table 46 -

| Stability data of epirubicin.HCl ready-to-use solution in 0.9% Sodium Chloride Injection, 4°C | | | |
|---|---|---|---|
| **Vials stored inverted** | | | |
| Batch Dosage | | INITIAL CONTROL | 6 months |
| TF/23274 10 mg | . | 2.218 | 2.247 |
| | .. | 100.0 | 101.3 |
| | ... | 2.7 | 4.1 |
| | .... | 2.9 | 2.9 |
| TF/23275 10 mg | . | 2.223 | 2.235 |
| | .. | 100.0 | 100.5 |
| | ... | 2.8 | 3.6 |
| | .... | 3.5 | 3.6 |
| 6001LA 10 mg | . | 2.155 | 2.219 |
| | .. | 100.0 | 103.0 |
| | ... | 1.9 | 3.5 |
| | .... | 3.0 | 3.0 |

. epirubicin.HCl assay (mg/ml)

.. epirubicin.HCl % initial

... related substances %

.... pH

Table 46 -   (continued)

| Stability data of epirubicin.HCl ready-to-use solution in 0.9% Sodium Chloride Injection, 4°C | | | |
|---|---|---|---|
| **Vials stored inverted** | | | |
| Batch Dosage | | INITIAL CONTROL | 6 months |
| 6001LB 20 mg | . | 1.961 | 1.955 |
| | .. | 100.0 | 99.7 |
| | ... | 1.2 | 2.8 |
| | .... | 3.1 | 3.1 |
| 6001LC 50 mg | . | 2.072 | 2.154 |
| | .. | 100.0 | 104.0 |
| | ... | 1.4 | 3.0 |
| | .... | 2.7 | 2.7 |

. epirubicin.HCl assay (mg/ml)

.. epirubicin.HCl % initial

... related substances %

.... pH

Table 47 -

| Stability data of epirubicin.HCl ready-to-use solution in 0.9% Sodium Chloride Injection, 8°C | | | | | |
|---|---|---|---|---|---|
| **Vials stored inverted** | | | | | |
| Batch Dosage | | INITIAL CONTROL | 1 months | 3 months | 6 months |
| TF/23274 10 mg | . | 2.218 | 2.223 | 2.139 | 2.227 |
| | .. | 100.0 | 100.2 | 96.4 | 100.4 |
| | ... | 2.7 | 2.5 | 3.9 | 3.7 |
| | .... | 2.9 | 2.8 | 2.9 | 2.9 |
| TF/23275 10 mg | . | 2.223 | 2.226 | 2.121 | 2.228 |
| | .. | 100.0 | 100.1 | 95.4 | 100.2 |
| | ... | 2.8 | 2.9 | 5.0 | 5.9 |
| | .... | 3.5 | 3.5 | 3.5 | 3.5 |
| 6001LA 10 mg | . | 2.155 | 2.220 | 2.156 | 2.215 |
| | .. | 100.0 | 103.0 | 100.0 | 102.8 |
| | ... | 1.9 | 3.6 | 3.3 | 3.6 |
| | .... | 3.0 | 3.0 | 3.0 | 3.0 |
| 6001LB 20 mg | . | 1.961 | 2.019 | 1.951 | 1.980 |
| | .. | 100.0 | 102.9 | 99.5 | 101.0 |
| | ... | 1.2 | 2.8 | 3.5 | 3.6 |
| | .... | 3.1 | 3.1 | 3.0 | 3.1 |

. epirubicin.HCl assay (mg/ml)

.. epirubicin.HCl % initial

... related substances %

.... pH

Table 47 -   (continued)

| Stability data of epirubicin.HCl ready-to-use solution in 0.9% Sodium Chloride Injection, 8°C | | | | | |
|---|---|---|---|---|---|
| **Vials stored inverted** | | | | | |
| Batch Dosage | | INITIAL CONTROL | 1 months | 3 months | 6 months |
| 6001LC 50 mg | . | 2.072 | 2.066 | 2.132 | 2.060 |
| | .. | 100.0 | 99.7 | 102.9 | 99.4 |
| | ... | 1.4 | 2.8 | 3.3 | 3.2 |
| | .... | 2.7 | 2.7 | 2.6 | 2.7 |

. epirubicin.HCl assay (mg/ml)
.. epirubicin.HCl % initial
... related substances %
.... pH

Table 48 -

| Stability date of epirubicin.HCl ready-to-use solution in 0.9% Sodium Chloride Injection, 15°C | | | | | |
|---|---|---|---|---|---|
| **Vials stored inverted** | | | | | |
| Batch Dosage | | INITIAL CONTROL | 1 months | 3 months | 6 months |
| TF/23274 10 mg | . | 2.218 | 2.235 | 2.173 | 2.081 |
| | .. | 100.0 | 100.8 | 98.0 | 93.8 |
| | ... | 2.7 | 4.1 | 3.9 | 5.4 |
| | .... | 2.9 | 2.9 | 2.9 | 2.9 |
| TF/23275 10 mg | . | 2.223 | 2.221 | 2.108 | n.d. |
| | .. | 100.0 | 99.9 | 94.8 | |
| | ... | 2.8 | 3.2 | 5.0 | |
| | .... | 3.5 | 3.5 | 3.4 | |
| 6001LA 10 mg | . | 2.155 | 2.210 | 2.121 | 2.058 |
| | .. | 100.0 | 102.5 | 98.4 | 96.8 |
| | ... | 1.9 | 3.4 | 3.5 | n.d. |
| | .... | 3.0 | 3.0 | 3.0 | 3.0 |
| 6001LB 20 mg | . | 1.961 | 2.009 | 1.915 | n.d. |
| | .. | 100.0 | 102.4 | 97.6 | |
| | ... | 1.2 | 3.6 | 3.7 | |
| | .... | 3.1 | 3.1 | 3.0 | |
| 6001LC 50 mg | . | 2.072 | 2.052 | 2.081 | 2.036 |
| | .. | 100.0 | 99.0 | 100.4 | 98.3 |
| | ... | 1.4 | 2.6 | 4.0 | 3.1 |
| | .... | 2.7 | 2.7 | 2.6 | 2.7 |

. epirubicin.HCl assay (mg/ml)
.. epirubicin.HCl % initial
... related substances %
.... pH
n.d. = not determined

Table 49 -

| Stability data of epirubitin.HCl ready-to-use solution in 0.9% Sodium Chloride Injection, 27°C | | | | |
|---|---|---|---|---|
| **Vials stored inverted** | | | | |
| Bath Dosage | | INITIAL CONTROL | 1 month | 3 months |
| TF/23274 10 mg | . | 2.218 | 2.111 | 1.941 |
| | .. | 100.0 | 95.2 | 87.5 |
| | ... | 2.7 | 5.4 | 7.1 |
| | .... | 2.9 | 2.9 | 2.9 |
| TF/23275 10 mg | . | 2.223 | 2.041 | 1.729 |
| | .. | 100.0 | 91.8 | 77.8 |
| | ... | 2.8 | 6.5 | 5.3 |
| | .... | 3.5 | 3.5 | 3.2 |
| 6001LA 10 ag | . | 2.155 | 2.144 | 1.938 |
| | .. | 100.0 | 99.5 | 89.9 |
| | ... | 1.9 | 5.2 | 9.9 |
| | .... | 3.0 | 3.0 | 3.0 |
| 6001LB 20 ag | . | 1.961 | 1.867 | 1.738 |
| | .. | 100.0 | 95.7 | 88.8 |
| | ... | 1.2 | 4.1 | 8.5 |
| | .... | 3.1 | 3.1 | 3.0 |
| 6001LC 50 mg | . | 2.072 | 2.009 | 1.866 |
| | .. | 100.0 | 96.9 | 90.1 |
| | ... | 1.4 | 4.1 | 9.1 |
| | .... | 2.7 | 2.7 | 2.6 |

. pirubicin.HCl assay (mg/l)
.. epirubicin.HCl % initial
... related substantes %
.... pH

Table 50 -

| Stability data of epirubicin.HCl ready-to-use solution in 0.9% Sodium Chloride Injection, 250 foot candles | | | | |
|---|---|---|---|---|
| **Vials stored inverted** | | | | |
| Batch Dosage | | INITIAL CONTROL | 1 month | 3 months |
| TF/23274 10 mg | . | 2.218 | 2.059 | 1.764 |
| | .. | 100.0 | 92.8 | 79.5 |
| | ... | 2.7 | 5.3 | 11.3 |
| | .... | 2.9 | 2.9 | 2.9 |

. epirubicin.HCl assay (mg/ml)
.. epirubicin.HCl % initial
... related substantes %
.... pH

Table 50 -   (continued)

| Stability data of epirubicin.HCl ready-to-use solution in 0.9% Sodium Chloride Injection, 250 foot candles | | | | | |
|---|---|---|---|---|---|
| **Vials stored inverted** | | | | | |
| Batch Dosage | | INITIAL CONTROL | 1 month | 3 months | |
| TF/23275 10 mg | . | 2.223 | 1.974 | 1.363 | |
| | .. | 100.0 | 88.8 | 61.3 | |
| | ... | 2.8 | 11.3 | 13.1 | |
| | .... | 3.5 | 3.5 | 3.1 | |
| 6001LA 10 mg | . | 2.155 | 1.984 | 1.667 | |
| | .. | 100.0 | 92.1 | 77.4 | |
| | ... | 1.9 | 5.0 | 12.6 | |
| | .... | 3.0 | 3.0 | 3.0 | |
| 6001LB 20 mg | . | 1.961 | 1.864 | 1.626 | |
| | .. | 100.0 | 95.0 | 82.9 | |
| | ... | 1.2 | 5.9 | 10.7 | |
| | .... | 3.1 | 3.0 | 2.9 | |
| 6001LC 50 mg | . | 2.072 | 2.118 | 2.002 | |
| | .. | 100.0 | 102.2 | 96.6 | |
| | ... | 1.4 | 2.6 | 5.9 | |
| | .... | 2.7 | 2.7 | 2.6 | |

. epirubicin.HCl assay (mg/ml)

.. epirubicin.HCl % initial

... related substantes %

.... pH

Table 51 -

| Stability data of epirubitin.HCl ready-to-use solution in 0.9% Sodium Chloride Injection,-20°C | | | | |
|---|---|---|---|---|
| **Vials stored inverted** | | | | |
| Batch Dosage | | INITIAL CONTROL | 1 month | 3 months |
| TF/23274 10 mg | . | 2.218 | | |
| | .. | 100.0 | n.d. | n.d. |
| | ... | 2.7 | | |
| | .... | 2.9 | | |
| TF/23273 10 mg | . | 2.223 | | |
| | .. | 100.0 | n.d. | n.d. |
| | ... | 2.8 | | |
| | .... | 3.5 | | |

. epirubicin.HCl assay (mg/ml)

.. epirubicin.HCl % initial

... related substantes %

.... pH n.d. = not determined

Table 51 -   (continued)

| Stability data of epirubitin.HCl ready-to-use solution in 0.9% Sodium Chloride Injection,-20°C | | | | |
|---|---|---|---|---|
| **Vials stored inverted** | | | | |
| Batch Dosage | | INITIAL CONTROL | 1 month | 3 months |
| 6001LA 10 mg | . | 2.155 | 2.185 | 2.131 |
| | .. | 100.0 | 101.4 | 98.9 |
| | ... | 1.9 | 2.8 | 3.0 |
| | .... | 3.0 | 3.0 | 3.0 |
| 6001LB 20 mg | . | 1.961 | 1.991 | 1.958 |
| | .. | 100.0 | 101.5 | 99.8 |
| | ... | 1.2 | 3.0 | 3.3 |
| | .... | 3.1 | 3.0 | 3.0 |
| 6001LC 50 mg | . | 2.072 | 2.049 | 2.184 |
| | .. | 100.0 | 98.9 | 105.4 |
| | ... | 1.4 | 2.9 | 3.3 |
| | .... | 2.7 | 2.7 | 2.7 |

. epirubicin.HCl assay (mg/ml)

.. epirubicin.HCl % initial

... related substantes %

.... pH
n.d. = not determined

Table 52 -

| Stability data of epirubicin.HCl ready-to-use solution in 5% Dextrose solution, 4°C | | | |
|---|---|---|---|
| **Vials stored upright** | | | |
| Batch Dosage | | INITIAL CONTROL | 6 months |
| TF/23270 10 mg | . | 2.178 | |
| | .. | 100.0 | n.d. |
| | ... | 3.7 | |
| | .... | 2.7 | |
| TF/23273 10 mg | . | 2.132 | |
| | .. | 100.0 | n.d. |
| | ... | 3.1 | |
| | .... | 3.5 | |
| 6001MA 10 mg | . | 2.131 | 2.173 |
| | .. | 100.0 | 102.0 |
| | ... | 2.0 | 4.0 |
| | .... | 3.0 | 3.0 |

. epirubicin.HCl assay (mg/ml)

.. epirubicin.BCl % initial

... related substances %

.... pH
n.d. = not determined

Table 52 -   (continued)

| Stability data of epirubicin.HCl ready-to-use solution in 5% Dextrose solution, 4°C | | | |
|---|---|---|---|
| **Vials stored upright** | | | |
| Batch Dosage | | INITIAL CONTROL | 6 months |
| 6001MB 20 mg | . | 1.973 | 1.917 |
| | .. | 100.0 | 97.5 |
| | ... | 1.7 | 4.0 |
| | .... | 3.1 | 3.1 |
| 6001MC 50 mg | . | 2.091 | 2.183 |
| | .. | 100.0 | 104.4 |
| | ... | 1.1 | 3.3 |
| | .... | 2.7 | 2.7 |

. epirubicin.HCl assay (mg/ml)

.. epirubicin.BCl % initial

... related substances %

.... pH
n.d. = not determined

Table 53

| Stability data of epirubicin.HCl ready-to-use solution in 5% Dextrose solution, 8°C | | | | | |
|---|---|---|---|---|---|
| **Vials stored upright** | | | | | |
| Batch Dosage | | INITIAL CONTROL | 1month | 3months | 6months |
| TF/23270 10 mg | . | 2.178 | | | |
| | .. | 100.0 | n.d. | n.d. | n.d. |
| | ... | 3.7 | | | |
| | .... | 2.9 | | | |
| TF/23273 10 mg | . | 2.132 | | | |
| | .. | 100.0 | n.d. | n.d. | n.d. |
| | ... | 3.1 | | | |
| | .... | 3.5 | | | |
| 6001MA 10 mg | . | 2.131 | 2.133 | 2.185 | 2.152 |
| | .. | 100.0 | 97.8 | 102.5 | 101.0 |
| | ... | 2.0 | 2.9 | 3.1 | 4.4 |
| | .... | 3.0 | 3.0 | 3.0 | 3.0 |
| 6001MB 20 mg | . | 1.973 | 1.979 | 1.936 | 1.934 |
| | .. | 100.0 | 100.3 | 98.1 | 98.4 |
| | ... | 1.7 | 2.2 | 3.3 | 3.3 |
| | .... | 3.1 | 3.1 | 3.0 | 3.1 |

. epirubicin.HCl assay (mg/ml)

... epirubicin.HCl % initial

... related substances %

... pH
n.d. = not determined

Table 53   (continued)

| Stability data of epirubicin.HCl ready-to-use solution in 5% Dextrose solution, 8°C | | | | | |
|---|---|---|---|---|---|
| **Vials stored upright** | | | | | |
| Batch Dosage | | INITIAL CONTROL | 1month | 3months | 6months |
| 6001MC 50 mg | . | 2.091 | 2.093 | 2.073 | 2.053 |
| | .. | 100.0 | 100.1 | 99.1 | 98.2 |
| | ... | 1.1 | 3.3 | 2.5 | 3.3 |
| | .... | 2.7 | 2.6 | 2.6 | 2.7 |

. epirubicin.HCl assay (mg/ml)

... epirubicin.HCl % initial

... related substances %

... pH n.d. = not determined

Table 54 -

| Stability data of epirubicin.HCl ready-to-use solution in 5% Dextrose solution, 4°C | | | |
|---|---|---|---|
| **Vials stored inverted** | | | |
| Batch Dosage | | INITIAL CONTROL | 6 months |
| TF/23270 10 mg | . | 2.178 | 2.171 |
| | .. | 100.0 | 99.7 |
| | ... | 3.7 | 4.3 |
| | .... | 2.7 | 2.7 |
| TF/23273 10 mg | . | 2.132 | 2.214 |
| | .. | 100.0 | 103.8 |
| | ... | 3.1 | n.d. |
| | .... | 3.5 | 3.5 |
| 6001MA 10 mg | . | 2.131 | 2.241 |
| | .. | 100.0 | 105.2 |
| | ... | 2.0 | 3.6 |
| | .... | 3.0 | 3.0 |
| 6001MB 20 mg | . | 1.973 | 1.899 |
| | .. | 100.0 | 96.6 |
| | ... | 1.7 | 2.9 |
| | .... | 3.1 | 3.1 |
| 6001MC 50 mg | . | 2.091 | 2.128 |
| | .. | 100.0 | 101.8 |
| | ... | 1.1 | 3.0 |
| | .... | 2.7 | 2.7 |

. epirubicin.HCl assay (mg/ml)

.. epirubicin.HCl % initial

... related substances %

.... pH

Table 55 -

| Stability data of epirubicin.HCl ready-to-use solution in 5% Dextrose solution, 8°C | | | | | |
|---|---|---|---|---|---|
| **Vials stored inverted** | | | | | |
| Batch Dosage | | INITIAL CONTROL | 1 month | 3 months | 6 months |
| TF/23270 10 mg | . | 2.178 | 2.207 | 2.201 | 2.228 |
| | .. | 100.0 | 101.3 | 101.0 | 102.3 |
| | ... | 3.7 | 3.9 | 3.8 | 2.6 |
| | .... | 2.7 | 2.7 | 2.7 | 2.7 |
| TF/23273 10 mg | . | 2.132 | 2.189 | 2.143 | 2.149 |
| | .. | 100.0 | 102.7 | 100.5 | 100.8 |
| | ... | 3.1 | 3.2 | 4.3 | 2.8 |
| | .... | 3.5 | 3.5 | 3.4 | 3.5 |
| 6001MA 10 mg | . | 2.131 | 2.184 | 2.179 | 2.227 |
| | .. | 100.0 | 100.1 | 99.9 | 104.4 |
| | ... | 2.0 | 3.2 | 3.0 | 3.6 |
| | .... | 3.0 | 3.0 | 3.0 | 3.0 |
| 6001MB 20 mg | . | 1.973 | 1.964 | 1.913 | 1.893 |
| | .. | 100.0 | 99.5 | 97.0 | 96.3 |
| | ... | 1.7 | 2.3 | 3.6 | 3.5 |
| | .... | 3.1 | 3.0 | 3.0 | 3.1 |
| 6001MC 50 mg | . | 2.091 | 2.060 | 2.078 | 2.161 |
| | .. | 100.0 | 98.5 | 99.4 | 103.3 |
| | ... | 1.1 | 2.9 | 2.9 | 3.5 |
| | .... | 2.7 | 2.6 | 2.6 | 2.7 |

. epirubitin.HCl assay (mg/ml)

.. epirubitin.HCl % initial

... related substances %

.... pH

Table 56 -

| Stability data of epirubicin.HCl ready-to-use solution in 5% Deztrose solution, 15°C | | | | | |
|---|---|---|---|---|---|
| **Vials stored inverted** | | | | | |
| Batch Dosage | | INITIAL CONTROL | 1 months | 3 months | 6 months |
| TF/23270 10 mg | . | 2.178 | 2.219 | 2.170 | 2.237 |
| | .. | 100.0 | 101.9 | 99.6 | 102.7 |
| | ... | 3.7 | 3.4 | 4.3 | 2.7 |
| | .... | 2.7 | 2.7 | 2.6 | 2.7 |

. spirubitin.HCl assay (mg/ml)

.. epirubicin.HCl % initial

... related substances %

.... pH

Table 56 -   (continued)

| Stability data of epirubicin.HCl ready-to-use solution in 5% Deztrose solution, 15°C | | | | | |
|---|---|---|---|---|---|
| **Vials stored inverted** | | | | | |
| Batch Dosage | | INITIAL CONTROL | 1 months | 3 months | 6 months |
| TF/23273 10 mg | . | 2.132 | 2.153 | 2.079 | 2.119 |
| | .. | 100.0 | 101.0 | 97.5 | 99.4 |
| | ... | 3.1 | 3.4 | 4.5 | n.d. |
| | .... | 3.5 | 3.5 | 3.4 | 3.4 |
| 6001MA 10 mg | . | 2.131 | 2.158 | 2.155 | 2.133 |
| | .. | 100.0 | 98.9 | 98.8 | 100.1 |
| | ... | 2.0 | 3.3 | 2.4 | 4.5 |
| | .... | 3.0 | 3.0 | 3.0 | 3.0 |
| 6001MB 20 mg | . | 1.973 | 1.978 | 1.893 | |
| | .. | 100.0 | 100.2 | 95.9 | n.d. |
| | ... | 1.7 | 2.0 | 3.7 | |
| | .... | 3.1 | 3.1 | 3.0 | |
| 6001MC 50 mg | . | 2.091 | 2.121 | 2.066 | 2.061 |
| | .. | 100.0 | 101.4 | 98.8 | 98.5 |
| | ... | 1.1 | 3.2 | 2.9 | 2.1 |
| | .... | 2.7 | 2.6 | 2.7 | 2.7 |

. spirubitin.HCl assay (mg/ml)

.. epirubicin.HCl % initial

... related substances %

.... pH

Table 57 -

| Stability data of epirubicin.HCl ready-to-use solution in 5% Dextrose solution, 27°C | | | | |
|---|---|---|---|---|
| **Vials stored inverted** | | | | |
| Batch Dosage | | INITIAL CONTROL | 1 month | 3 months |
| TF/23270 10 mg | . | 2.178 | 2.113 | 2.052 |
| | .. | 100.0 | 97.0 | 94.2 |
| | ... | 3.7 | 4.7 | 4.7 |
| | .... | 2.7 | 2.7 | 2.7 |
| TF/23273 10 mg | . | 2.132 | 2.068 | 1.944 |
| | .. | 100.0 | 97.0 | 91.2 |
| | ... | 3.1 | 6.0 | 7.5 |
| | .... | 3.5 | 3.5 | 3.3 |

. epirubicin.HCl assay (mg/ml)

.. epirubicin.HCl % initial

... related substances %

.... pH

Table 57 -   (continued)

| Stability data of epirubicin.HCl ready-to-use solution in 5% Dextrose solution, 27°C | | | | |
|---|---|---|---|---|
| **Vials stored inverted** | | | | |
| Batch Dosage | | INITIAL CONTROL | 1 month | 3 months |
| 6001MA 10 mg | . | 2.131 | 2.144 | 2.033 |
| | .. | 100.0 | 96.9 | 93.2 |
| | ... | 2.0 | 3.2 | 7.3 |
| | .... | 3.0 | 3.0 | 3.0 |
| 6001MB 20 mg | . | 1.973 | 1.919 | 1.796 |
| | .. | 100.0 | 97.3 | 91.1 |
| | ... | 1.7 | 2.1 | 6.2 |
| | .... | 3.1 | 3.0 | 3.0 |
| 6001MC 50 mg | . | 2.091 | 2.096 | 1.896 |
| | .. | 100.0 | 100.6 | 90.7 |
| | ... | 1.1 | 3.0 | 6.6 |
| | .... | 2.7 | 2.6 | 2.6 |

. epirubicin.HCl assay (mg/ml)

.. epirubicin.HCl % initial

... related substances %

.... pH

Table 58 -

| Stability data of epirubicin.HCl ready-to-use solution in 5% Dextrose solution, 250 foot-candles | | | | |
|---|---|---|---|---|
| **Vials stored inverted** | | | | |
| Batch Dosage | | INITIAL CONTROL | 1 months | 3 months |
| TF/23270 10 mg | . | 2.178 | 2.132 | 2.003 |
| | .. | 100.0 | 97.9 | 92.0 |
| | ... | 3.7 | 6.7 | 9.2 |
| | .... | 2.7 | 2.7 | 2.7 |
| TF/23273 10 mg | . | 2.132 | 2.046 | 1.796 |
| | .. | 100.0 | 96.0 | 84.2 |
| | ... | 3.1 | 5.8 | 10.3 |
| | .... | 3.5 | 3.4 | 3.2 |
| 6001MA 10 mg | . | 2.131 | 2.079 | 1.988 |
| | .. | 100.0 | 95.3 | 91.1 |
| | ... | 2.0 | 3.7 | 8.2 |
| | .... | 3.0 | 3.0 | 3.0 |

. epirubitin.HCl assay (mg/ml)

.. epirubitin.HCl % initial

... related substantes %

.... pH

Table 58 -   (continued)

| Stability data of epirubicin.HCl ready-to-use solution in 5% Dextrose solution, 250 foot-candles | | | | |
|---|---|---|---|---|
| **Vials stored inverted** | | | | |
| Batch Dosage | | INITIAL CONTROL | 1 months | 3 months |
| 6001MB 20 mg | . | 1.973 | 1.894 | 1.803 |
| | .. | 100.0 | 96.0 | 91.4 |
| | ... | 1.7 | 2.0 | 5.6 |
| | .... | 3.1 | 3.0 | 3.0 |
| 6001MC 50 mg | . | 2.091 | 2.022 | 1.988 |
| | .. | 100.0 | 96.7 | 95.1 |
| | ... | 1.1 | 4.0 | 4.2 |
| | .... | 2.7 | 2.6 | 2.6 |

. epirubitin.HCl assay (mg/ml)

.. epirubitin.HCl % initial

... related substantes %

.... pH

Table 59 -

| Stability data of epirubitin.HCl ready-to-use solution in 5% Dextrose solution, -20°C | | | | |
|---|---|---|---|---|
| **Vials stored inverted** | | | | |
| Batch Dosage | | INITIAL CONTROL | 1 month | 3 months |
| TF/23270 10 mg | . | 2.178 | | |
| | .. | 100.0 | n.d. | n.d. |
| | ... | 3.7 | | |
| | .... | 2.7 | | |
| TF/23273 10 mg | . | 2.132 | | |
| | .. | 100.0 | n.d. | n.d. |
| | ... | 3.1 | | |
| | .... | 3.5 | | |
| 6001MA 10 mg | . | 2.131 | 2.168 | 2.162 |
| | .. | 100.0 | 99.4 | 99.1 |
| | ... | 2.0 | 2.2 | 3.3 |
| | .... | 3.0 | 3.0 | 3.0 |
| 6001MB 20 mg | . | 1.973 | 1.985 | 1.994 |
| | .. | 100.0 | 100.6 | 101.4 |
| | ... | 1.7 | 2.6 | 3.8 |
| | .... | 3.1 | 3.0 | 3.0 |

. epirubicin.HCl assay (mg/ml)

.. epirubitin.HCl % initial

... related substances %

.... pH n.d. = not determined

Table 59 -   (continued)

| Stability data of epirubitin.HCl ready-to-use solution in 5% Dextrose solution, -20°C | | | | |
|---|---|---|---|---|
| **Vials stored inverted** | | | | |
| Batch Dosage | | INITIAL CONTROL | 1 month | 3 months |
| 6001MC | . | 2.091 | 2.114 | 2.090 |
| 50 mg | .. | 100.0 | 101.9 | 99.9 |
| | ... | 1.1 | 2.5 | 2.6 |
| | .... | 2.7 | 2.6 | 2.6 |

. epirubicin.HCl assay (mg/ml)

.. epirubitin.HCl % initial

... related substances %

Table 60 -

| Stability data of epirubicin.HCl ready-to-use solution in Water for Injection Batch TF/23176 | | | | | | | | |
|---|---|---|---|---|---|---|---|---|
| Vials stored upright | | | | | | | | |
| Storage temperature | Tests | Time (months) | | | | | | |
| | | 0 | 1 | 2 | 3 | 6 | 9 | 12 |
| - 20°C | . | 2.079 | 2.061 | | 2.059 | | | |
| | .. | 100.0 | 99.1 | n.d. | 99.0 | n.d. | n.d. | |
| | ... | 4.0 | 3.8 | | 4.9 | | | |
| | .... | 3.0 | 3.1 | | 3.2 | | | |
| 4°C | . | 2.079 | | | 2.062 | 2.061 | | 1.976 |
| | .. | 100.0 | n.d. | n.d. | 99.2 | 99.1 | n.d. | 95.0 |
| | ... | 4.0 | | | 3.7 | 3.4 | | 6.7 |
| | .... | 3.0 | | | 3.2 | 3.2 | | 3.1 |
| 8°C | . | 2.079 | 2.041 | 2.043 | 1.995 | 1.986 | | 1.950 |
| | .. | 100.0 | 98.2 | 98.3 | 96.0 | 95.5 | n.d. | 93.8 |
| | ... | 4.0 | 3.6 | 3.8 | 4.0 | 3.4 | | 6.7 |
| | .... | 3.0 | 3.0 | 3.0 | 3.1 | 3.2 | | 3.1 |
| 15°C | . | 2.079 | 2.001 | 1.989 | 1.9% | 1.923 | | 1.757 |
| | .. | 100.0 | 96.2 | 95.7 | 95.7 | 92.5 | n.d. | 84.5 |
| | ... | 4.0 | 3.7 | 3.9 | 3.9 | 3.5 | | 8.8 |
| | .... | 3.0 | 3.0 | 3.0 | 3.1 | 3.2 | | 3.1 |
| 27°C | . | 2.079 | 1.977 | 1.879 | 1.830 | 1.480 | | |
| | .. | 100.0 | 95.1 | 90.4 | 88.0 | 71.2 | n.d. | |
| | ... | 4.0 | 5.0 | 6.8 | 6.9 | 9.0 | | |
| | .... | 3.0 | 3.0 | 3.1 | 3.1 | 3.1 | | |

. epirubicin.HCl assay (mg/ml)

.. epirubicin.HCl % initial

... related substances %

.... pH n.d. = not determined

Table 61 -

| Stability data of epirubicin.HCl ready-to-use solution in Water for Injection Batch TF/23176 | | | | | | | | |
|---|---|---|---|---|---|---|---|---|
| Vials stored inverted | | | | | | | | |
| Storage temperature | Tests | Time (months) | | | | | | |
| | | 0 | 1 | 2 | 3 | 6 | 9 | 12 |
| - 20°C | . | 2.079 | 2.052 | | 2.060 | | | |
| | .. | 100.0 | 98.7 | n.d. | 99.1 | n.d. | n.d. | n.d. |
| | ... | 4.0 | 3.7 | | 4.6 | | | |
| | .... | 3.0 | 3.1 | | 3.2 | | | |
| 4°C | . | 2.079 | | | 2.045 | 2.067 | | |
| | .. | 100.0 | n.d. | n.d. | 98.4 | 99.4 | n.d. | n.d. |
| | ... | 4.0 | | | 3.6 | 2.9 | | |
| | .... | 3.0 | | | 3.2 | 3.2 | | |
| 8°C | . | 2.079 | 2.033 | 2.039 | 2.022 | 1.977 | | |
| | .. | 100.0 | 97.8 | 98.1 | 97.3 | 95.1 | n.d. | n.d. |
| | ... | 4.0 | 3.5 | 3.1 | 3.7 | 3.1 | | |
| | .... | 3.0 | 3.0 | 3.0 | 3.1 | 3.2 | | |
| 15°C | . | 2.079 | 1.992 | 1.994 | 1.994 | 1.934 | | 1.759 |
| | .. | 100.0 | 95.8 | 95.9 | 95.9 | 93.0 | n.d. | 84.6 |
| | ... | 4.0 | 3.6 | 3.9 | 4.6 | 4.4 | | 8.6 |
| | .... | 3.0 | 3.0 | 3.0 | 3.1 | 3.2 | | 3.1 |
| 27°C | . | 2.079 | 1.964 | 1.836 | 1.779 | 1.568 | | |
| | .. | 100.0 | 94.5 | 88.3 | 85.6 | 75.4 | | |
| | ... | 4.0 | 5.0 | 7.9 | 6.9 | 7.4 | | |
| | .... | 3.0 | 3.0 | 3.1 | 3.1 | 3.1 | | |
| R.T.+ 100 F.C. | . | 2.079 | 1.923 | | 1.778 | | | |
| | .. | 100.0 | 92.5 | n.d. | 85.5 | | | |
| | ... | 4.0 | 4.8 | | 7.5 | | | |
| | .... | 3.0 | 3.1 | | 3.1 | | | |
| R.T.+ 250 F.C. | . | 2.079 | 1.886 | | 1.493 | | | |
| | .. | 100.0 | 90.7 | n.d. | 71.8 | | | |
| | ... | 4.0 | 6.6 | | 13.5 | | | |
| | .... | 3.0 | 3.0 | | 3.0 | | | |

. epirubicin.HCL assay (mg/ml)

.. epirubicin.HCl % initial

... related substances %

.... pH n.d. = not determined R.T. = room temperature F.C. = foot candles

## Claims

1. A storage stable, sterile, pyrogen-free, injectable anthracycline glycoside solution which consists essentially of a physiologically acceptable salt of an anthracycline glycoside dissolved in a physiologically acceptable solvent therefor, and which has not been reconstituted from a lyophilizate; **characterised in that** the pH of the solution has been adjusted to from 2.5 to 4.0.

2. A solution according to claim 1 in a sealed container.

3. A solution according to claim 1 or 2 wherein the anthracycline glycoside is doxorubicin or 4'-epi-doxorubicin.

4. A solution according to any one of the preceding claims having a pH of from 2.5 to 3.7.

5. A solution according to claim 4, wherein the pH is from 2.6 to 3.5.

6. A solution according to any one of the preceding claims containing dextrose, lactose, sorbitol or mannitol as a stabilising and/or tonicity adjustment agent.

7. A solution according to any one of the preceding claims, wherein the physiologically acceptable solvent is water or physiological saline or an aqueous 5% dextrose solution.

8. A solution according to any one of the preceding claims having a pH of about 3.

9. A process for producing the storage-stable, sterile, pyrogen-free, injectable solution of any one of the preceding claims, which process comprises

(i) dissolving the physiologically acceptable salt, which salt is not in the form of a lyophilizate, in the physiologically acceptable solvent therefor;
(ii) optionally, adding one or more formulation adjuvants selected from co-solubilizing agents, stabilizing agents, tonicity adjustment agents, preservatives and pharmaceutically acceptable chelating agents; and
(iii) adding a physiologically acceptable acid or buffer to adjust the pH to from 2.5 to 4.0 as desired; the process being effected in such a manner that the resultant solution is sterile and pyrogen-free.

10. A process according to claim 9 wherein the solution is passed through a sterilising filter after step (iii).